(19) Europäisches Patentamt
European Patent Office
Office européen des brevets

(11) Veröffentlichungsnummer : **0 110 831**
**B1**

(12) # EUROPÄISCHE PATENTSCHRIFT

(45) Veröffentlichungstag der Patentschrift : **04.03.87**

(51) Int. Cl.⁴ : **C 07 D335/16**, G 03 C 1/68, G 03 C 1/71, G 03 F 7/26

(21) Anmeldenummer : **83810540.1**

(22) Anmeldetag : **21.11.83**

(54) **Neue durch alpha-Aminoalkylgruppen substituierte Thioxanthone.**

(30) Priorität : **25.11.82 CH 6873/82**

(43) Veröffentlichungstag der Anmeldung : **13.06.84 Patentblatt 84/24**

(45) Bekanntmachung des Hinweises auf die Patenterteilung : **04.03.87 Patentblatt 87/10**

(84) Benannte Vertragsstaaten : **CH DE FR GB LI NL**

(56) Entgegenhaltungen :
**EP-A- 0 010 897**
**EP-A- 0 011 918**
**CH-A- 508 620**
**DE-A- 2 344 799**
**DE-A- 3 117 568**
**GB-A- 1 359 278**
**CHEMICAL ABSTRACTS, Band 86, No. 5, 31. Jänner 1977, Columbus, Ohio, USA PROTIVA: "Tranquilizong thioxanthene derivative" Seite 349, Spalte 2 Abstract No. 29617t**

(73) Patentinhaber : **CIBA-GEIGY AG**
**Klybeckstrasse 141**
**CH-4002 Basel (CH)**

(72) Erfinder : **Finter, Jürgen, Dr.**
**Zaslusstrasse 100**
**D-7800 Freiburg (DE)**
Erfinder : **Fischer, Walter, Dr.**
**Vogesenstrasse 77**
**CH-4153 Reinach (CH)**

**EP 0 110 831 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, durch α-Aminoalkylgruppen substituierte Thioxanthone, Verfahren zu deren Herstellung und die dabei erhältlichen neuen Zwischenprodukte, sowie die Verwendung der neuen Thioxanthone, besonders in lichtempfindlichen, zu Kondensations- oder Additionsreaktionen befähigten Stoffgemischen.

In der DE-A1-3 117 568 sind unter anderem 3-Aminoxanthon-1-carbonsäurealkylester beschrieben, die als Initiatoren oder Sensibilisatoren für die photochemische Vernetzung von äthylenisch ungesättigten Verbindungen oder Polyolefinen geeignet sind. Aus den Stoffgemischen können Reliefabbildungen nach dem Negativverfahren erzeugt werden.

Es wurden neue wertvolle, durch α-Aminoalkylgruppen substituierte Thioxanthone der Formel I

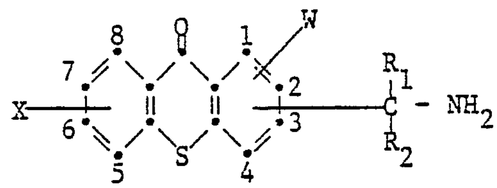

(I)

gefunden, worin

X Wasserstoff, Chlor, Brom, $C_{1-4}$-Alkyl oder $C_{1-6}$-Alkoxy,
$R_1$ Wasserstoff, $C_{1-6}$-Alkyl, Phenyl, $-COOCH_3$ oder $-COOC_2H_5$ und
$R_2$ Wasserstoff, $C_{1-6}$-Alkyl, $-COOCH_3$ oder $-COOC_2H_5$ bedeuten oder
$R_1$ und $R_2$ zusammen $-(CH_2)_e-$ mit e = 4 oder 5 darstellen und
W Wasserstoff oder $-COOC_{1-4}$-Alkyl bedeutet.

Chlor- oder Bromatome, Alkyl- oder Alkoxygruppen X sind vorzugsweise in 7-Stellung gebunden.

Stellen X, $R_1$ oder $R_2$ Alkylgruppen dar, so kann es sich dabei um geradkettige oder verzweigte Gruppen handeln, wie z. B. Methyl, Aethyl, n-Propyl, Isopropyl, n-Butyl. sek.-Butyl, tert-Butyl-, n-Pentyl, 2- oder 3-Pentyl, n-Hexyl und 2-Aethylhexyl. Alkylgruppen X, $R_1$ und $R_2$ sind vorzugsweise geradkettig und weisen insbesondere 1 oder 2 C-Atome auf. Bedeutet W eine Gruppe $-COOC_{1-4}$-Alkyl, so können die Alkylgruppen in diesen Resten ebenfalls geradkettig oder verzweigt sein, wobei Alkylgruppen der oben erwähnten Art in Betracht kommen. Bevorzugt sind $-COOCH_3$ und $-COOC_2H_5$.

Als Alkoxygruppen X kommen geradkettige oder verzweigte Gruppen, wie die Methoxy-, Aethoxy-, n-Propoxy-, Isopropoxy-, n-Butoxy-, sek-Butoxy-, n-Pentyloxy- und n-Hexyloxygruppe in Betracht. Bevorzugt handelt es sich um geradkettige Alkoxygruppen mit 1-4 und vor allem 1 oder 2 C-Atomen.

$R_1$ und $R_2$ stellen bevorzugt gleiche Gruppen dar.

Die Gruppe $-C(R_1)(R_2)-NH_2$ ist vorzugsweise in 2-, 3- oder 4-Stellung gebunden. Bevorzugt sind Verbindungen der Formel I, worin X Wasserstoff oder in 7-Stellung gebundenes Chlor oder Methyl bedeutet, die Gruppe $-C(R_1)(R_2)-NH_2$ in 2-, 3- oder 4-Stellung gebunden ist, $R_1$ und $R_2$ unabhängig voneinander Wasserstoff, Methyl, Aethyl, $-COOCH_3$ oder $-COOC_2H_5$ und W Wasserstoff, $-COOCH_3$ oder $-COOC_2H_5$ bedeuten. Besonders bevorzugt sind Verbindungen der Formel I, worin X Wasserstoff oder in 7-Stellung gebundenes Methyl ist, die Gruppe $-C(R_1)(R_2)-NH_2$ in 2- oder 3-Stellung gebunden ist. $R_1$ und $R_2$ je Wasserstoff oder Methyl und W Wasserstoff, $-COOCH_3$ oder $-COOC_2H_5$ bedeuten. Ganz besonders bevorzugt sind das 2-Aminomethylthioxanthon, 3-(2-Amino-2-propyl)-thioxanthon, 3-(2-Amino-2-propyl)-7-methylthioxanthon und der 3-(2-Amino-2-propyl)-thioxanthon-1-carbonsäureäthylester.

Die Verbindungen der Formel I können nach an sich bekannten Methoden hergestellt werden, z. B. wie folgt :

a) Verbindungen der Formel I, worin $R_1$ ungleich Wasserstoff ist : Durch Umsetzung einer Verbindung der Formel II

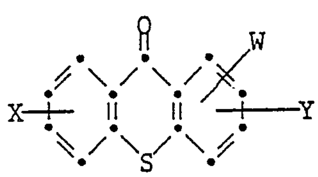

(II)

mit einer Verbindung der Formel III

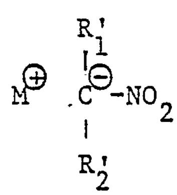

(III)

zu einer Verbindung der Formel IV

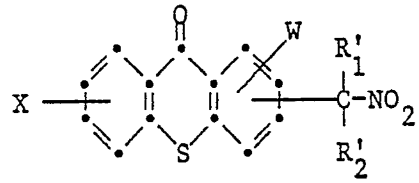

(IV)

und Reduktion der Verbindung der Formel IV zu Verbindungen der Formel I, worin $R_1$ ungleich Wasserstoff ist. Dieses Verfahren eignet sich besonders zur Herstellung von Verbindungen der Formel I, worin die Gruppe $—C(R_1)(R_2)—NH_2$ in 1-Stellung und vor allem in 3-Stellung gebunden ist.

b) Verbindungen der Formel I, worin $R_1$ und $R_2$ unabhängig voneinander $—COOCH_3$ oder $—COOC_2H_5$ sind :

Durch Umsetzung einer Verbindung der Formel II in Gegenwart einer Base mit einer Verbindung der Formel Va oder Vb

$$HC(R_1'')(R_2'')—NH_2 \qquad (Va)$$

oder

$$HC(R_1'')(R_2'')—NHCOCH_3 \qquad (Vb)$$

zu einer Verbindung der Formel Ia oder VI

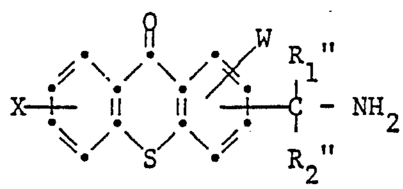

oder

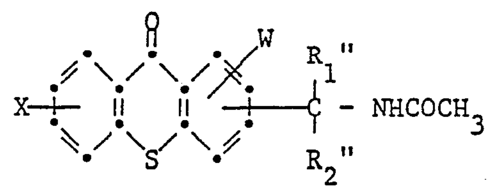

(Ia) (VI)

und Hydrolyse der Verbindungen der Formel VI zu Verbindungen der Formel Ia, worin $R_1''$ und $R_2''$ unabhängig voneinander $—COOCH_3$ oder $—COOC_2H_5$ darstellen, bevorzugt aber gleich sind.

c) Verbindungen der Formel I, worin $R_1$ oder $R_1$ und $R_2$ Wasserstoff sind :

Durch Umsetzung einer Verbindung der Formel VII

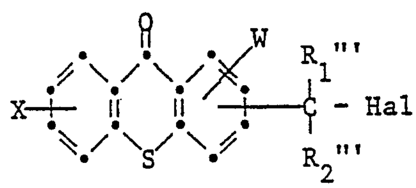

(VII)

mit einem Azid der Formel VIII

$$M_1^{q+} (N_3^-)_q \qquad (VIII)$$

zu einer Verbindung der Formel IX

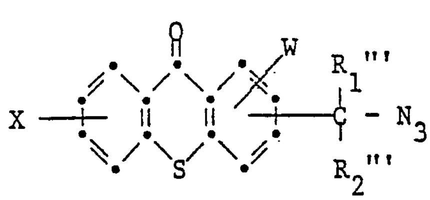

(IX)

und Reduktion der Verbindungen der Formel IX zu Verbindungen der Formel I, worin $R_1$ oder $R_1$ und $R_2$ Wasserstoff sind.

Dabei haben $R_1$, $R_2$, X und W die unter Formel I angegebene Bedeutung, Y stellt Brom, Chlor, Fluor oder $—NO_2$ dar. $R'_1$ und $R'_2$ haben dieselbe Bedeutung wie $R_1$ und $R_2$, wobei $R'_1$ aber ungleich Wasserstoff ist, $R''_1$ und $R''_2$ bedeuten unabhängig voneinander $—COOCH_3$ oder $—COOC_2H_5$, $R'''_1$ ist Wasserstoff und $R'''_2$ hat dieselbe Bedeutung wie $R_2$ oder $R'''_1$ und $R'''_2$ sind beide Wasserstoff, Hal stellt ein Halogenatom, besonders Brom oder Chlor dar, $M^+$ ist das Kation einer organischen oder anorganischen Base, q ist 1 oder 2 und $M_1^+$ bedeutet ein Alkalimetall-, Erdalkalimetall- oder quaternäres Ammoniumkation.

Die Umsetzung der Verbindungen der Formel II mit den Salzen der Formel III wird zweckmässig in Gegenwart eines inerten organischen Lösungsmittels durchgeführt. Als solche eignen sich z. B. Dialkylsulfoxide, wie Dimethyl- und Diäthylsulfoxid, N,N-Dialkylamide von aliphatischen Monocarbonsäuren mit 1-3 C-Atomen im Säureteil, wie N,N-Dimethylformamid und N,N-Dimethylacetamid, cyclische Amide, wie N-Methylpyrrolidon, sowie Hexamethylphosphorsäuretriamid.

Die Salze der Formel III können als solche eingesetzt oder *in situ* aus einer Verbindung $HC(R_1)(R_2)—NO_2$ und einer geeigneten organischen oder anorganischen Base gebildet werden. $M^+$ stellt z. B. ein Alkalimetall-, Erdalkalimetall- oder Ammoniumkation dar, wie ein Trialkyl-, Benzyltrialkyl- oder Tetraalkylammoniumkation mit je 1-12 und besonders je 1-4 C-Atomen in den Alkylgruppen. Bevorzugt stellt $M^+$ ein Alkalimetallkation, besonders das Natrium- oder Kaliumkation dar. Wird das Salz der Formel III *in situ* gebildet, so können z. B. tertiäre Amine, wie Triäthylamin, oder quaternäre Ammoniumsalze, wie Tetramethyl-, Teträthyl-, Benzyltrimethyl- und Benzyltriäthylammoniumsalze, Alkalimetall- oder Erdalkalimetallcarbonate, -hydroxide oder -halogenide, wie Natrium- und Kaliumcarbonat, Natrium- und Kaliumhydroxid, Natrium-, Kalium- oder Lithiumfluorid, verwendet werden. Bevorzugt ist die Bildung der Salze der Formel III *in situ*, besonders unter Verwendung von Natrium- oder Kaliumcarbonat.

Die Reduktion der Verbindungen der Formel IV zu den Verbindungen der Formel I kann auf an sich bekannte Weise vorgenommen werden, z. B. in Gegenwart von HCl oder Essigsäure und Eisen, Essigsäure und Zink, unter Rückflussbedingungen, oder katalytisch, besonders in Gegenwart von Platin- oder Palladiumkatalysatoren, und eines inerten organischen Lösungsmittels, z. B. Dioxan, N,N-Dimethylformamid, Methanol oder Aethanol.

Als Basen für die Umsetzung der Verbindungen der Formel II mit den Verbindungen der Formel Va oder Vb eignen sich z. B. Natrium- oder Kaliumcarbonat. Die Hydrolyse der Verbindungen der Formel VI zu Verbindungen der Formel Ia erfolgt zweckmässig in saurem Medium, z. B. unter Zusatz von konz. HCl.

Stellt $M_1^+$ in Formel VIII ein quaternäres Ammoniumkation dar, so handelt es sich z. B. um ein Tetralkyl- oder Benzyltrialkylammoniumkation mit je 1-12 und vor allem 1-4 C-Atomen in den Alkylgruppen, insbesondere das Tetramethyl- oder Trimethylbenzylammoniumkation. Beispiele von geeigneten Alkalimetall- oder Erdalkalimetall- aziden der Formel VIII sind das Lithium-, Natrium-, Kalium-, Calcium-, Magnesium- und Bariumazid. Bevorzugt verwendet man Alkalimetallazide, besonders das Natriumazid.

Die Umsetzung der Verbindungen der Formel VII mit den Aziden der Formel VIII wird zweckmässig in Gegenwart eines inerten organischen Lösungsmittel, wie N,N-Dimethylformamid, N,N-Dimethylacetamid, Dimethylsulfoxid, N-Methylpyrrolidon oder Hexamethylphosphorsäuretriamid, durchgeführt. Bevorzugte Lösungsmittel sind Dimethylsulfoxid, N,N-Dimethylformamid und N,N-Dimethylacetamid.

Die Reduktion (Hydrierung) der Verbindungen der Formel IX zu Verbindungen der Formel I kann auf an sich bekannte Weise vorgenommen werden. Geeignete Reduktionsmittel sind z. B. komplexe Hydride, wie Natriumborhydrid und Lithiumaluminiumhydrid, Hydrazin oder Alkalimetallsulfide. Bevorzugt ist die katalytische Hydrierung, wobei bekannte Hydrierungskatalysatoren eingesetzt werden können. Besonders geeignet sind Edelmetall-Katalysatoren, wie Platin-, Rhodium-, Palladium-, Ruthenium- oder Iridiumkatalysatoren. Besonders bevorzugt sind Platin/Kohle- und Palladium/Kohle-Katalysatoren.

Das 2-Aminomethylthioxanthon kann auch nach folgendem Reaktionsschema hergestellt werden :

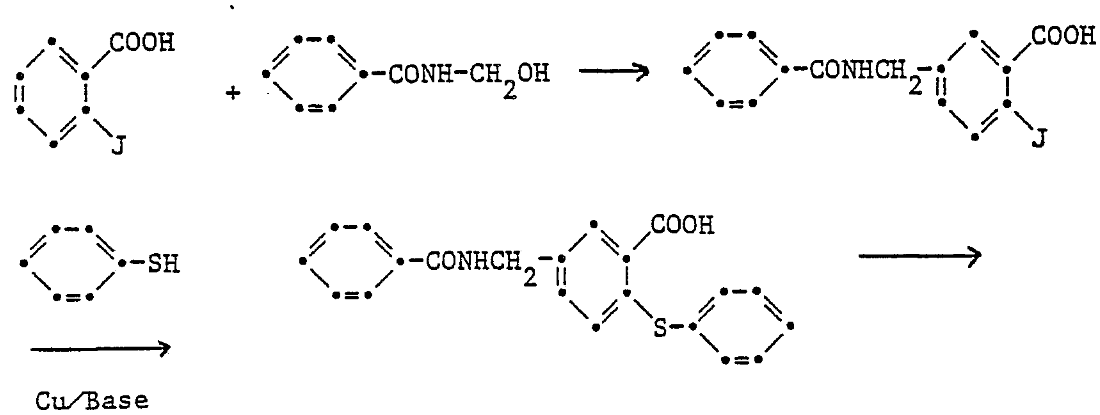

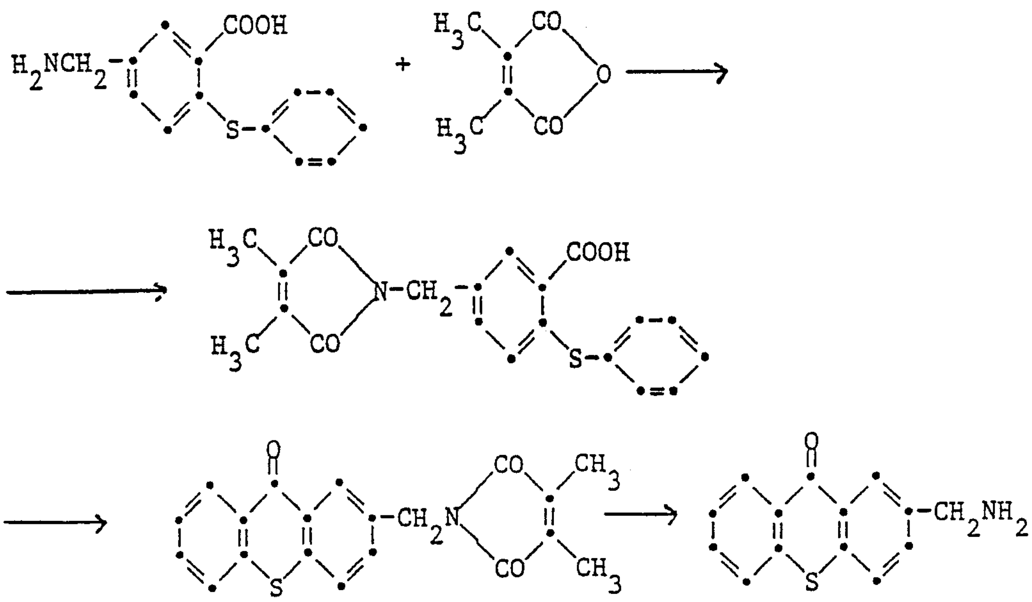

Schliesslich können die Zwischenprodukte der Formel IV auch durch Nitrierung von Verbindungen der Formel X

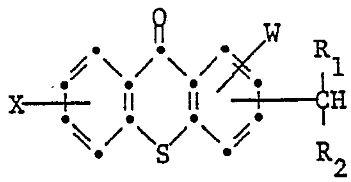

(X)

mit Salpetersäure hergestellt werden. Dabei tritt allerdings z. T. auch eine Nitrierung der Benzolringe ein. Die erhaltenen Stoffgemische können z. B. durch Chromatographie aufgetrennt werden. In Formel X haben X, W, $R_1$ und $R_2$ die unter Formel I angegebene Bedeutung.

Die zur Herstellung der Verbindungen der Formel I entwickelten Zwischenprodukte der Formel IV mit W = Wasserstoff und die Zwischenprodukte der Formel IX sind neu und ebenfalls Gegenstand vorliegender Erfindung. Dabei gilt in Bezug auf bevorzugte Bedeutungen von X und W und bevorzugte Stellungen der Gruppe $-C(R'''_1)(R'''_2)-N_3$ oder $-C(R_1')(R_2')-NO_2$ das oben Angegebene. $R_1'''$ und $R_2'''$ sind vorzugsweise je Wasserstoff.

Die Ausgangsprodukte der Formeln II, III, IV mit W = $-COOC_{1-4}$-Alkyl, Va, Vb, VII, VIII und X sind bekannt oder können nach an sich bekannten Methoden hergestellt werden. Verbindungen der Formel II und X können z. B. analog den in der DE-OS 31 17 568-A 1 beschriebenen Verfahren erhalten werden, während sich Verbindungen der Formel VII gemäss Rev. Chim. (Bukarest), *19*, 561 (1968) herstellen lassen.

Die Verbindungen der Formel I finden z. B. Anwendung in lichtempfindlichen Stoffgemischen, vor allem zur Bilderzeugung. Gegenstand der Erfindung sind somit auch neue zu Kondensations- oder Additionsreaktionen befähigte lichtempfindliche und gegebenenfalls vernetzbare Stoffgemische, enthaltend

1) ein Thioxanthon der Formel I,

2) eine oder mehrere Verbindungen ausgewählt aus Di- bis Polyglycidyläthern von Phenol- und Kresolnovolacken und Verbindungen der Formeln XI bis XIII

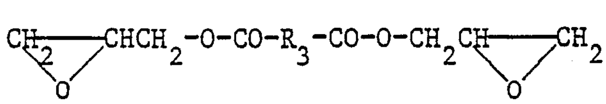

(XI)

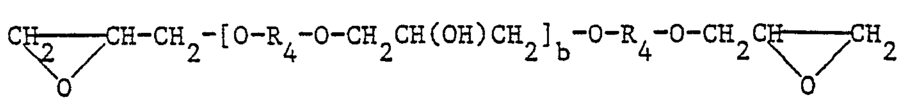

(XII)

und

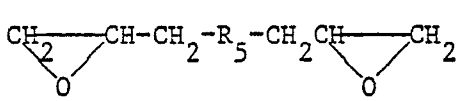

(XIII)

und gegebenenfalls Verbindungen der Formeln XIV, XV und/oder XVI

$$HO—R_6—O—[CO—R_3—CO—O—R_6—O]_a—H \quad (XIV),$$

$$HO—[Y_3—O]_o—Y_3—OH \quad (XV)$$

und/oder

$$HOOC—R_3—CO—[O—R_6—O—CO—R_3—CO]_a—OH \quad (XVI),$$

wobei der Anteil an Verbindungen der Formeln XIV, XV und/oder XVI höchstens 80 Mol.%, bezogen auf alle unter 2) genannten Reaktionskomponenten beträgt,

3) gegebenenfalls ein Vernetzungsmittel und

4) gegebenenfalls ein Salz eines Metalls der Gruppe Ib oder VIII des Periodischen Systems, worin

a eine Zahl von 1-100, besonders 2-50,

b eine Zahl von 0-150, besonders 0,1-150 und vor allem 2-100,

$R_3$ die direkte Bindung, $—C_mH_{2m}—$ mit m = 2-12 oder Cyclohexylen, Cyclohexenylen, Phenylen oder Endomethylencyclohexenylen, die durch eine Methylgruppe substituiert sein können,

$R_4$ $—C_mH_{2m}—$ mit m = 2-12, Phenylen,

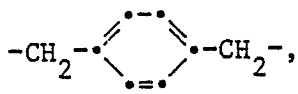

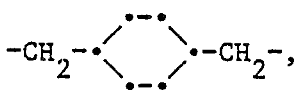

eine Gruppe der Formeln

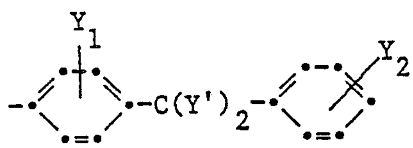

oder $—(Y_3O)_o—Y_3—$

Y' Wasserstoff oder Methyl,

$Y_1$ und $Y_2$ unabhängig voneinander Wasserstoff, Chlor oder Brom,

$Y_3$ $—(CH_2)_2—$, $—CH_2CH(CH_3)—$ oder $—(CH_2)_4—$,

o eine Zahl von 1-50,

$R_5$

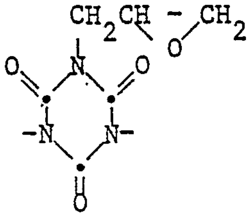 , 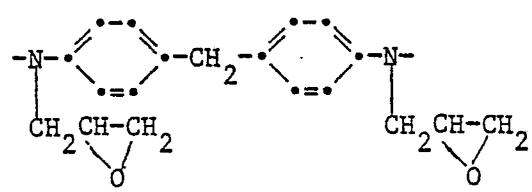

den Rest des Aethylenharnstoffs, 1,3-Propylenharnstoffs, 5,5-Dimethylhydantoins, 2-Hydroxyäthyl-5,5-dimethylhydantoins oder 2-Hydroxypropyl-5,5-dimethylhydantoins und

$R_6$ $—C_mH_{2m}—$ mit m = 2-12, $—(CH_2CH_2O)_r—CH_2CH_2—$ mit r = 1-40, besonders 1-20, $—CH(CH_3)CH_2OCH_2CH(CH_3)—$, $—CH_2—C(CH_3)_2—OCO—C(CH_3)_2CH_2—$, Cyclohexylen,

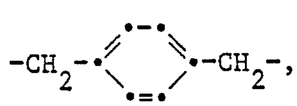 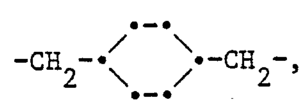

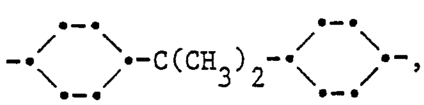 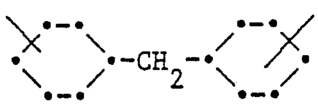

Naphthylen, Biphenylen oder gegebenenfalls durch eine Methyl-, Methoxy- oder Nitrogruppe substituiertes Phenylen bedeuten.

Die erfindungsgemässen Stoffgemische müssen definitionsgemäss zu Kondensations- oder Additionsreaktionen befähigt sein. Die fakultativ einzusetzenden Verbindungen der Formeln XIV und XV können z. B. mit den Di- bis Polyglycidyläthern von Phenol- oder Kresolnovolacken oder den Verbindungen der Formeln XI bis XIII und XVI reagieren.

Der Anteil an Verbindungen der Formel I beträgt zweckmässig zwischen 1 und 60 Mol.%, vorzugsweise 2 und 45 Mol.%, bezogen auf die unter 2) angegebenen Verbindungen. Sind a oder b grösser als 1, so können die einzelnen $R_3$, $R_4$, $R_6$ und $Y_3$ in den Verbindungen der Formeln XII und XIV bis XVI (Oligomere bzw. Polymere) gleiche oder unterschiedliche Bedeutungen haben, und wiederkehrende Strukturelemente in solchen Oligomeren oder Polymeren können statistisch oder blockartig angeordnet sein.

Durch $R_3$, $R_4$ oder $R_6$ dargestellte Gruppen $-C_mH_{2m}-$ können geradkettig oder verzweigt sein. Als Beispiele solcher Gruppen seien genannt:

$-(CH_2)_2-$, $-CH_2CH(CH_3)-$, $-(CH_2)_3-$, $-(CH_2)_4-$, $-(CH_2)_2-C(CH_3)_2-(CH_2)_2-$, $-(CH_2)_6-$, $-C(CH_3)_2-$, $-CH_2C(CH_3)_2-CH_2-CH(CH_3)(CH_2)_2-$, $-CH_2CH(CH_3)CH(CH_3)CH_2CH(CH_3)CH_2-$, $-(CH_2)_7-$, $-(CH_2)_8-$, $-(CH_2)_{10}-$ und $-(CH_2)_{12}-$.

Stellen $R_4$ und/oder $R_6$ Gruppen $-C_mH_{2m}-$ dar, so handelt es sich insbesondere um Reste des Aethylenglykols, 1,4-Butandiols, Neopentylglykols oder 1,6-Hexandiols. Ist $R_3$ eine Gruppe $-C_mH_{2m}-$, so kommen vor allem Reste der Bernsteinsäure-, Adipinsäure, Pimelinsäure, Azelainsäure oder Sebacinsäure in Betracht.

Bedeutet $R_6$ Cyclohexylen, so handelt es sich insbesondere um den Rest des 1,2-Cyclohexandiols. Als Cyclohexylengruppen $R_3$ kommen vor allem 1,3- und insbesondere 1,4-Cyclohexylen in Betracht, die durch Methyl substituiert sein können, bevorzugt aber unsubstituiert sind.

Stellt $R_6$ Naphthylen, Biphenylen oder gegebenenfalls durch eine Methyl-, Methoxy- oder Nitrogruppe substituiertes Phenylen dar, so kommen z. B. Reste des 1,4-, 1,6-, 1,8- und 2,6-Dihydroxynaphthalins, des 2,2'-Biphenyls, Resorcins, 2,5-Dihydroxyanisols, 1,2-Dihydroxy-4-nitrobenzols, 2,5- und 3,4-Dihydroxytoluols in Betracht. Bevorzugt bedeutet $R_6$ $-(CH_2)_2-$, $-(CH_2)_4-$, $-(CH_2)_6-$, $-CH_2CH_2OCH_2CH_2-$, $-C(CH_3)_2-$,

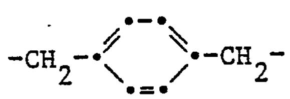

oder 1,3-Phenylen.

Bedeutet $R_3$ gegebenenfalls durch Methyl substituiertes Cyclohexenylen, Phenylen oder Endomethylencyclohexenylen, so handelt es sich beispielsweise um Reste der Methyl-tetrahydrophthalsäure, Endomethylentetrahydrophthalsäure, Tetrahydrophthalsäure, Phthalsäure, Isophthalsäure oder Terephthalsäure. $R_3$ ist bevorzugt $-(CH_2)_m-$ mit m = 2-10, 1,3- oder 1,4-Phenylen, 1,3- oder 1,4-Cyclohexylen.

Ist $R_4$ Phenylen, so handelt es sich insbesondere um 1,3-Phenylen.

Bedeutet $R_4$ eine Gruppe der Formel

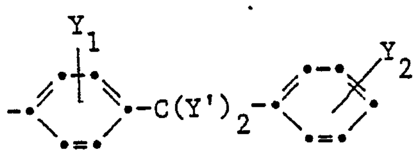

so stellen $Y_1$ und $Y_2$ vorzugsweise je in 2,2'-Stellung gebundenes Chlor oder Brom dar. Besonders bevorzugt sind jedoch solche Gruppen, worin $Y_1$ und $Y_2$ Wasserstoff bedeuten. Stellt $R_4$ eine gruppe $-(Y_3O)_o-Y_3$ dar, so ist $Y_3$ bevorzugt $-(CH_2)_2-$ oder $-CH_2CH(CH_3)-$ und o stellt insbesondere 1 bis 40, vor allem 2-20, dar.

$R_4$ ist bevorzugt $-C_mH_{2m}-$ mit m = 2,4 oder 6 oder

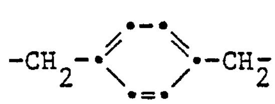

vor allem jedoch eine Gruppe der Formeln

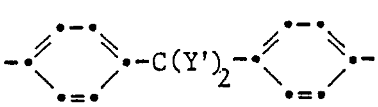

$-(CH_2CH_2O)_o-CH_2CH_2-$ und/oder $-[CH_2CH(CH_3)O]_o-CH_2CH(CH_3)-$ mit Y' = Wasserstoff und vor allem Methyl und o = 1 bis 40, vor allem 2-20.

$R_5$ ist vorzugsweise der Rest des 5,5-Dimethylhydantoins, 2-Hydroxyäthyl- oder 2-Hydroxypropyl-5,5-dimethylhydantoins oder des Triglycidylisocyanurats.

Bevorzugt sind Stoffgemische, die ein Thioxanthon der Formel I, eine oder mehrere definitionsgemässe Verbindungen mit Glycidylengruppen und gegebenenfalls eine Verbindung der Formel XVI, sowie gegebenenfalls ein Vernetzungsmittel und/oder ein definitionsgemässes Metallsalz enthalten, wobei X Wasserstoff oder in 7-Stellung gebundenes Chlor oder Methyl bedeutet, die Gruppe $-C(R_1)(R_2)-NH_2$ in 2-, 3- oder 4-Stellung gebunden ist, $R_1$ und $R_2$ unabhängig voneinander Wasserstoff, Methyl, Aethyl, $-COOCH_3$ oder $-COOC_2H_5$ und W Wasserstoff, $-COOCH_3$ oder $-COOC_2H_5$ bedeuten. Besonders bevorzugt sind Gemische, die eine oder mehrere definitionsgemässe Verbindungen mit Glycidylengruppen und ein Thioxanthon der Formel I, sowie gegebenenfalls ein Vernetzungsmittel und/oder ein definitionsgemässes Metallsalz enthalten, worin X Wasserstoff oder in 7-Stellung gebundenes Methyl ist, die Gruppe $-C(R_1)(R_2)-NH_2$ in 2- oder 3-Stellung gebunden ist, $R_1$ und $R_2$ je Wasserstoff oder Methyl und W Wasserstoff, $-COOCH_3$ oder $-COOC_2H_5$ bedeuten.

Besonders bevorzugt sind Gemische, die eine Verbindung der Formel I, worin X Wasserstoff oder in 7-Stellung gebundenes Methyl ist, die Gruppe $-C(R_1)(R_2)-NH_2$ in 2- oder 3-Stellung gebunden ist, $R_1$ und $R_2$ je H oder Methyl und W Wasserstoff, $-COOCH_3$ oder $-COOC_2H_5$ bedeuten, eine oder mehrere Verbindungen ausgewählt aus Di- und/oder Triglycidyläthern von Phenol- oder Kresolnovolacken, Triglycidylisocyanurat, Hexahydrophthalsäurediglycidylester, N,N'-Diglycidyl-5,5-dimethylhydantoin, N-Glycidyl-N'-2-oxyäthylglycidyl- und/oder N-Glycidyl-N'-2-oxy-propylglycidyl-5,5-dimethylhydantoin und Verbindungen der Formeln A), B) und C)

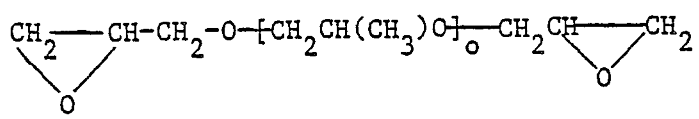 (A)

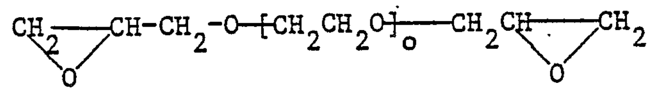 (B)

und

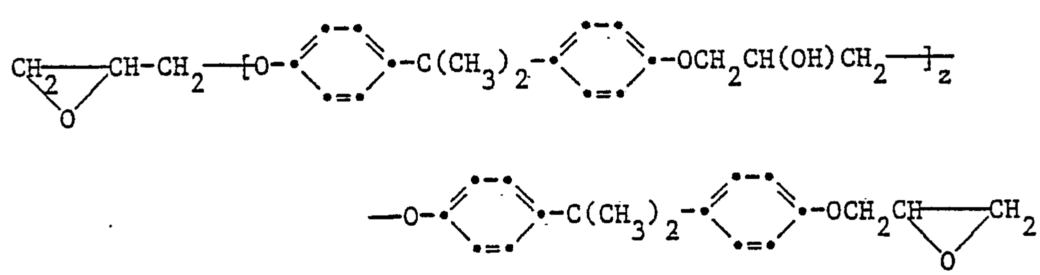 (C)

sowie gegebenenfalls ein Vernetzungsmittel und/oder ein definitionsgemässes Metallsalz enthalten, wobei o 2-40 und insbesondere 2-20 und z 0,1-13, vor allem 2-11, bedeuten.

Ganz besonders bevorzugt sind Gemische, die als Verbindung der Formel I 2-Aminomethylthioxanthon, 3-(2-Amino-2-propyl)-thioxanthon, 3-(2-Amino-2-propyl)-7-methylthioxanthon oder 3-(2-Amino-2-propyl)-thioxanthon-1-carbonsäureäthylester, eine Verbindung der Formel A) oder B) im Gemisch mit einer Verbindung der Formel C) und gegebenenfalls ein Vernetzungsmittel und/oder ein definitionsgemässes Metallsalz enthalten, oder Gemische, die als Verbindung der Formel I 2-Aminomethylthioxanthon, 3-(2-Amino-2-propyl)-thioxanthon, 3-(2-Amino-2-propyl)-7-methylthioxanthon oder 3-(2-Amino-2-propyl)-thioxanthon-1-carbonsäureäthylester, N,N'-Diglycidyl-5,5-dimethylhydantoin, N-Glycidyl-N'-2-oxyäthylglycidyl- und/oder N-Glycidyl-N'-2-oxypropylglycidyl-5,5-dimethylhydantoin im Gemisch mit einer Verbindung der Formel C) und gegebenenfalls ein Vernetzungsmittel und/oder ein definitionsgemässes Metallsalz enthalten wobei o 2-20 und z 2-11 bedeuten.

Gegenstand der Erfindung sind auch die lichtempfindlichen, gegebenenfalls vernetzten Reaktionsprodukte, die dadurch erhältlich sind, dass man gegebenenfalls in Gegenwart eines Vernetzungsmittels eine Verbindung der Formel I mit einer oder mehreren Verbindungen ausgewählt aus Di- bis Polyglycidyläthern von Phenol- und Kresolnovolacken und Verbindungen der Formeln XI bis XIII und gegebenenfalls Verbindungen der Formeln XIV, XV und/oder XVI zur Reaktion bringt, wobei für den Anteil an Verbindungen der Formeln XIV bis XVI das oben Angegebene gilt, und die erhaltenen Reaktionsprodukte gegebenenfalls anschliessend mindestens teilweise mit einem Salz eines Metalls der Gruppe Ib oder VIII des Periodischen Systems komplexiert.

Bevorzugt sind Reaktionsprodukte, die dadurch erhältlich sind, dass man Gemische der oben angegebenen bevorzugten Art in an sich bekannter Weise zur Reaktion bringt. Werden mehrere

Verbindungen der unter 2) definierten Art eingesetzt, so kann die Umsetzung durch vorgängige (Poly)Addition oder (Poly)-Kondensation auch stufenweise durchgeführt werden, indem z. B. das Thioxanthon der Formel I zuerst mit (einem Unter- oder Ueberschuss) einer ersten definitionsgemässen Reaktionskomponente umgesetzt wird und man das erhaltene Reaktionsprodukt anschliessend gegebenenfalls in Gegenwart eines Vernetzungsmittels und/oder eines definitionsgemässen Metallsalzes mit der bzw. den weiteren Reaktionskomponente(n) zur Reaktion bringt. Andererseits ist es auch möglich, zuerst verschiedene Verbindungen der unter 2) genannten Art miteinander zur Reaktion zu bringen und das erhaltene Reaktionsprodukt in einer zweiten Stufe mit dem Thioxanthon der Formel I umzusetzen.

Je nach Art der Reaktionskomponenten und der Reaktionsreihenfolge und abhängig davon, welche Reaktionskomponenten im Ueber- bzw. Unterschuss eingesetzt werden, können unterschiedliche Verknüpfungen des Thioxanthons mit den unter 2) genannten Verbindungen bzw. mit diesen letztgenannten Verbindungen untereinander erzielt werden. So können beispielsweise Polymere, die wiederkehrende Strukturelemente der Formeln XVII oder XVIII

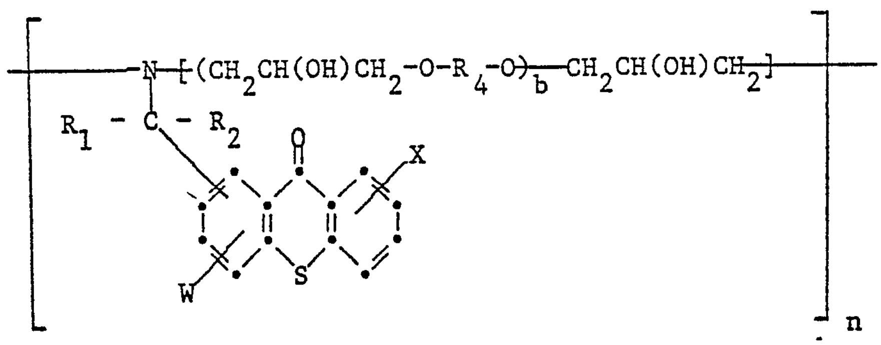

(XVII)

oder

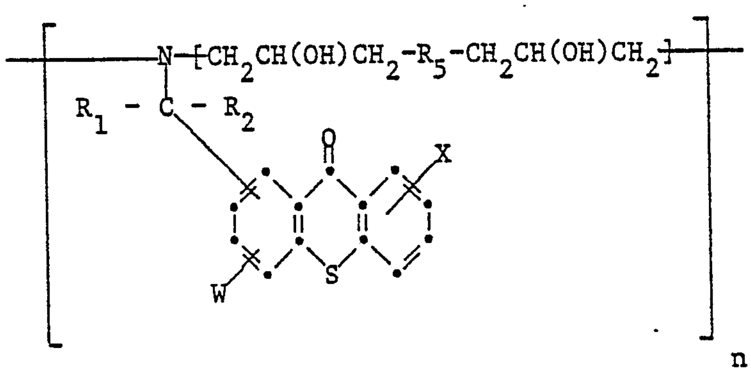

(XVIII)

aufweisen, oder Verbindungen der Formeln XIX oder XX

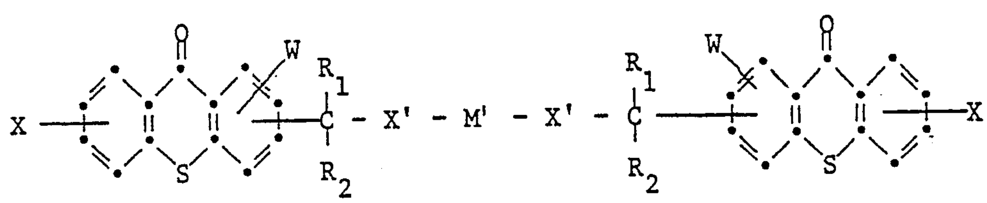

(XIX)

oder

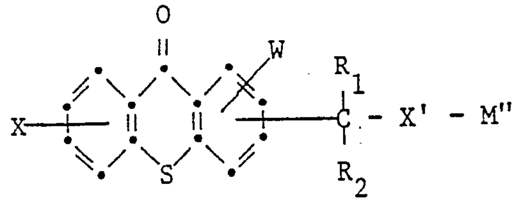

(XX)

hergestellt werden, die mindestens teilweise mit Metallionen eines Metalls der Gruppe Ib oder VIII des Periodischen Systems komplexiert sein können, worin $R_1$, $R_2$, X und W die unter Formel I angegebene Bedeutung haben,

X' —NH— oder $-\overset{|}{N}-$ darstellt,

M' gleiche oder verschiedene Reste von Di- bis Polyglycidyläthern von Phenol- oder Kresolnovolacken oder Gruppierungen der Formeln XIa, XIIa oder XIIIa

$$-CH_2CH(OH)CH_2-O-CO-R_3-CO-O-CH_2CH(OH)CH_2- \quad \text{(XIa),}$$

$$-CH_2CH(OH)CH_2-[O-R_4-O-CH_2CH(OH)CH_2]_b-O-R_4-O-CH_2CH(OH)CH_2- \quad \text{(XIIa)}$$

oder

$$-CH_2CH(OH)CH_2-R_5-CH_2CH(OH)CH_2- \quad \text{(XIIIa)}$$

und bei X' = —NH— gegebenenfalls teilsweise eine Gruppierung der Formel XVIa

$$-OC-R_3-CO-[O-R_6-O-CO-R_3-CO]_a- \quad \text{(XVIa)}$$

darstellt,

M" gleiche oder verschiedene Gruppierungen der Formel XIIb

$$-CH_2CH(OH)CH_2-[O-R_4-O-CH_2CH(OH)CH_2]_{b'}-O-R_4-O-CH_2\underset{\diagdown_{O}\diagup}{CH-CH_2} \quad \text{(XIIb)}$$

und bei X' = —NH— gegebenenfalls teilweise eine Gruppierung der Formeln XVIb

$$-OC-R_3-CO-[O-R_6-O-CO-R_3-CO]_{a'}-OH \quad \text{(XVIb)}$$

darstellt,

a' eine Zahl von 5-100 und b' eine Zahl von 5-150 darstellen und

a, b, X, W und $R_1$ bis $R_6$ die oben angegebene Bedeutung haben.

Lineare Polymere der oben angegebenen Formeln (X' ungleich $-\overset{|}{N}-$) weisen bevorzugt ein durchschnittliches Molekulargewicht von 600 bis 500 000 und insbesondere von 2000 bis 150 000 Dalton auf. Das durchschittliche Molekulargewicht kann nach an sich bekannten Methoden bestimmt werden, z. B. mittels Osmometrie oder Lichtstreuung.

Vernetzte Produkte können unter Umständen auch ohne Zusatz von Vernetzungsmitteln erhalten werden, z. B. bei der Umsetzung von Verbindungen der Formeln XI bis XIII mit den Thioxanthonen der Formel I.

Die Komplexierung kann vor, nach oder bevorzugt während der Applikation der Stoffgemische bzw. der daraus erhältlichen Reaktionsprodukte erfolgen. Bei der Herstellung von erfindungsgemässen Polymeren können auch Ausgangspolymere eingesetzt werden, die definitionsgemässe Metallsalze enthalten.

Für die Komplexierung eignen sich Salze der definitionsgemässen Metalle mit organischen oder anorganischen Säuren oder Gemische davon, wie Carboxylate, z. B. Formiate, Acetate, Stearate, Gluconate und Citrate; Halogenide, Nitrate, Sulfate und Perchlorate. Als Beispiele seien genannt: Eisen(III)acetat, -citrat, -gluconat, -nitrat, -sulfat und -perchlorat; Eisen(II)- oder Eisen(III)chlorid, Eisen(II)-oxalat; Ruthenium(III)chlorid; Kobalt(II)acetat, -nitrat oder -sulfat; Kobalt(II)chlorid oder -bromid; Rhodium(II)acetat, Rhodium(III)chlorid; Nickel(II)acetat, Nickel(II)bromid und -chlorid, Nickel(II)sulfat; Palladium(II)chlorid und -jodid, Palladiumacetat und -nitrat; Kupfer(II)formiat und -acetat, Kupfer(I)- und -(II)chlorid, -bromid und -jodid, Kupfer(II)nitrat oder -sulfat; Silberacetat, -chlorid, -bromid, -nitrat oder -sulfat. Bevorzugt sind Salze von Nicht-Edelmetallen, vor allem Eisen-, Kobalt-, Nickel- oder Kupfersalze. Ganz besonders bevorzugt sind Kupfersalze bzw. $Cu^{++}$-Ionen. Bevorzugt für die Komplexierung sind Kupfer(II)carboxylate und Kupferhalogenide. Ganz besonders bevorzugt verwendet man Kupfer(II)acetat oder Gemische aus Kupfer(II)acetat und Kupfer(II)-bromid im molaren Verhältnis von 9 : 1. Der Grad der Kompexierung beträgt vorzugsweise bis zu 15 %, bezogen auf die komplexierbaren Gruppen des Polymeren bzw. der Ausgangsprodukte. Komplexierbare Gruppen sind z. B. OH—, NH— oder sekundäre Aminogruppen, wie $N(CH_3)_2$-Gruppen.

Als Vernetzungsmittel kommen je nach Art der vorhandenen funktionellen Gruppen z. B. zwei- oder höherwertige Alkohole, Phenole oder Amine, Di-, Tri- oder Tetracarbonsäuren oder Derivate davon, wie Anhydride, in Betracht. Als Beispiele geeigneter mehrfunktioneller Verbindungen seien genannt: Diole $HO-R_4-OH$ oder $HO-R_6-OH$, Dicarbonsäuren $HOOC-R_3-COOH$, Oligoester der Formel XVI mit einem

durchschnittlichen Molekulargewicht von 300-6000 Dalton, sowie Diamine der Formel $H_2N—R_7—NH_2$. Dabei haben $R_3$, $R_4$ und $R_6$ die oben angegebene Bedeutung und $R_7$ stellt $—C_mH_{2m}—$ mit m = 2-12-Cyclohexylen, Naphthylen, gegebenenfalls durch eine Methyl-, Methoxy- oder Nitrogruppe substituiertes Phenylen, 1,3- oder 1,4-Xylylen, den Rest des 4,4'-Diaminodicyclohexylmethans, 4,4'-Diaminodiphenylmethans, 4,4'-Diaminodiphenyläthers, 4,4'-Diaminodiphenylsulfons oder Isophorondiamins dar. $R_7$ als Rest $—C_mH_{2m}—$ ist bevorzugt $—(CH_2)_2—$, Trimethylen, Tetramethylen, Hexamethylen, $—CH_2CH(CH_3)CH(CH_3)CH_2CH(CH_3)CH_2—$ oder $—CH_2C(CH_3)_2CH_2CH(CH_3)CH_2CH_2—$.

Ist $R_7$ Naphthylen oder gegebenenfalls durch eine Methyl-, Methoxy- oder Nitrogruppe substituiertes Phenylen, so kommen z. B. die folgenden Reste in Betracht : 1,2-, 1,3- und 1,4-Phenylen, 4-Methoxy-1,3-phenylen, 2-Nitro-1,4-phenylen, o- und m-Tolylen, 1,5- und 1,8-Naphthylen. Vorzugsweise stellt $R_7$ $—C_mH_{2m}—$ mit m = 2-10, 1,3- oder 1,4-Phenylen oder den Rest des 4,4'-Diaminophenylmethans, 4,4'-Diamindiphenyläthers oder Isophorondiamins dar. Als Vernetzungsmittel können ferner verwendet werden : Glycerin, Tris-(hydroxymethyl)-äthan und -propan, Penaerythrit, Diäthylentriamin, Triäthylentetramin, Bernsteinsäureanhydrid, Glutarsäureanhydrid, Phthalsäureanhydrid, Tetrahydro- und Hexahydrophthalsäureanhydrid, Trimellitsäureanhydrid, Pyromellitsäureanhydrid und Benzophenontetracarbonsäuredianhydride. Für die Vernetzung von OH— und/oder glycidylgruppenhaltigen Verbindungen verwendet man vorzugsweise Carbonsäureanhydride, wie Hexahydrophthalsäureanhydrid oder Phthalsäureanhydrid, bzw. zwei- oder mehrwertige Alkohole. Bevorzugt ist die Vernetzung von glycidylgruppenhaltigen Verbindungen mit Carbonsäureanhydriden oder zweiwertigen Alkoholen, besonders Hexahydrophthalsäureanhydrid oder Bisphenol A.

Die Kondensations- bzw. ringöffnenden Additionsreaktionen werden zweckmässig in Gegenwart eines inerten organischen Lösungsmittels bei Temperaturen zwischen 90 und 160 °C, bevorzugt 100 und 130 °C, vorgenommen. Als Lösungsmittel eignen sich z. B. Chlorbenzol, Dichlorbenzole, N,N-Dialkylamide von aliphatischen Monocarbonsäuren mit 1-3 C-Atomen im Säureteil, wie N,N-Dimethylformamid und N,N-Dimethylacetamid, Aethylenglykolmonomethyl- und -monoäthyläther, N-Methylpyrrolidon, Aethylenglykoldimethyl- oder -diäthyläther. Gegebenenfalls kann die Umsetzung in Gegenwart eines Katalysators, wie N,N-Dimethylbenzylamin, vorgenommen werden.

Die erfindungsgemässen lichtempfindlichen Stoffgemische bzw. Reaktionsprodukte finden z. B. Anwendung als Sensibilisatoren (Redox-Katalysatoren) bei verschiedenen Oxidations-/Reduktionsreaktionen oder als Beschichtungsmaterialien, z. B. für den Korrosionsschutz von Photohalbleiterdioden oder Halbleiterlaser. Besonders eignen sie sich aber zur Bilderzeugung durch Lichteinwirkung auf verschiedenen anorganischen oder organischen Substraten. Geeignete Substrate für die Bilderzeugung sind z. B. Glas, Metalle und Metalloxide, wie Aluminium, Aluminiumoxid und Kupfer, Keramik, Papier und hochmolekulare organische Materialien. Als hochmolekulare organische Materialien kommen natürliche und synthetische Polymere in Betracht, z. B. Cellulosematerialien, wie Celluloseacetate, Cellulosepropionate, Cellulosebutyrate und Celluloseäther, wie Methylcellulose ; von α,β-ungesättigten Säuren abgeleitete Polymere, wie Polyacrylate und Polymethacrylate, Polyacrylamide und Polyacrylnitril ; Styrolpolymere und Copolymere davon, z. B. Styrol/Butadiencopolymere und Acrylnitril/Butadien/Styrolcopolymere ; Vinyl- und Vinylidenpolymere und Copolymere davon, wie Polyvinylchlorid, Polyvinylidenchlorid, Vinylchlorid/Vinylidenchloridcopolymere, Vinylchlorid/Vinylacetatcopolymere ; von ungesättigten Alkoholen und Aminen abgeleitete Polymere und Derivate davon, wie Polyvinylalkohol, Polyvinylacetat und Polyallylmelamin ; vernetzte Epoxidharze ; Polyacetale ; Polyalkylenoxide und Polyphenylenoxide ; Polyamide, Polyimide, Polyamid/Polyimid-Blockcopolymere, Polysulfone und Polyester ; Alkydharze, z. B. Glyzerin/Phthalsäureharze und deren Mischungen mit Melamin/Formaldehydharzen, Melamin-Formaldehyd-, Harnstoff-Formaldehyd- und Phenol-Formaldehydharze.

Die erfindungsgemässen Stoffgemische und Reaktionsprodukte, insbesondere die Polymeren finden vor allem Anwendung zur Erzeugung von elektrisch leitenden Ueberzügen oder Mustern, besonders gedruckten Schaltungen. Zu diesem Zweck werden in an sich bekannter Weise unter Lichteinwirkung die Metallionen in den Stoffgemischen oder den mindestens teilweise komplexierten Reaktionsprodukten zu nullwertigen nichtleitenden Metallkeimen (nicht-leitenden sichtbaren Bildstellen) reduziert oder es werden bei Stoffgemischen oder Reaktionsprodukten, die keine Metallsalze bzw. Metallionen enthalten, Radikale erzeugt, auf denen dann in üblicher Weise durch stromlose Abscheidung von Metallen, wie Kupfer und Nickel, elektrisch leitende metallische Ueberzüge oder Muster hergestellt werden können. Diese metallischen Ueberzüge oder Muster können gewünschtenfalls durch elektrolytische Metallabscheidung mit konventionellen Metallabscheidungsbädern verstärkt werden. Für die Belichtung der erfindungsgemässen Stoffgemische oder Reaktionsprodukte können an sich beliebige geeignete Lichtquellen eingesetzt werden, z. B. Xenonlampen, Metallhalogenidlampen und besonders Quecksilberhoch- und -mitteldrucklampen.

## Beispiel 1

6 g (18,22 mMol) 3-Nitrothioxanthon-1-carbonsäureäthylester, 8,12 g (91,1 mMol) 2-Nitropropan, 7,55 g (54,66 mMol) Kaliumcarbonat und 20 ml N,N-Dimethylformamid (DMF) werden während 2 Stunden bei 50 °C gerührt. Die Mischung wird am Rotationsverdampfer eingeengt. Der Rückstand wird mit Salzäure auf pH 6 gestellt und mit Methylenchlorid extrahiert. Die organischen Extrakte werden mit gesättigter

NaCl-Lösung gewaschen, über Natriumsulfat getrocknet und eingedampft. Durch Umkristallisation aus Toluol werden 5,95 g (88 % d. Th.) 3-(2-Nitro-2-propyl)-thioxanthon-1-carbonsäureäthylester erhalten ; Smp. 198-201 °C.

Analyse für $C_{19}H_{17}NO_5S$ (Molgewicht 371,41) :
berechnet : C 61,45 H 4,62 N 3,77 O 21,54 S 8,63 %
gefunden : C 61,52 H 4,54 N 4,02 O 24,73 S 8,62 %.

3,0 g (8,07 mMol) 3-(2-Nitro-2-propyl)-thioxanthon-1-carbonsäureäthylester werden mit 1 g Raney-Nickel in 90 ml Dioxan bei 25 °C und 4 bar während 24 Stunden hydriert. Das Gemisch wird filtriert, und die Mutterlauge wird eingeengt. Der Rückstand wird in 50 ml Tetrahydrofuran/Toluol (1 : 1) gelöst und mit 2N HCl-Lösung extrahiert. Die HCl-Extrakte werden abgetrennt, mit $NaHCO_3$-Lösung auf pH 7-8 gestellt und mit Tetrahydrofuran/Toluol extrahiert. Die organischen Extrakte werden mit gesättigter NaCl-Lösung gewaschen, über Natriumsulfat getrocknet und eingedampft. Durch Trocknen im Hochvakuum werden 2,3 g (83 % d. Th.) teilweise kristalliner 3-(2-Amino-2-propyl)-thioxanthon-1-carbonsäureäthylester erhalten.

Analyse für $C_{19}H_{19}NO_3S$ (Molgewicht 341,43) :
berechnet : C 66,84 H 5,61 N 4,10 O 14,06 S 9,39 %
gefunden : C 67,53 H 6,32 N 3,87 O 13,87 S 8,52 %.

Analog wird aus 3-Nitrothioxanthon das 3-(2-Nitro-2-propyl)-thioxanthon (Smp. 131-133 °C, Ausbeute 40 %) erhalten, das wie oben zu 3-(2-Amino-2-propyl)-thioxanthon umgesetzt wird, Smp. 265-270°, Ausbeute 51 %.

Analyse für $C_{16}H_{15}NOS$ (Molgewicht 269, 36) :
berechnet : C 71,35 H 5,61 N 5,20 O 5,94 S 11,90 %
gefunden : C 70,64 H 5,72 N 5,17 O 6,24 S 11,62 %

Analog wird aus 3-Nitro-7-Methylthioxanthon das 3-(2-Nitro-2-propyl)-7-methyl-thioxanthon (Smp. 170-173 °C, Ausbeute 64 %) erhalten, das wie oben zu 3-(2-Amino-2-propyl)-7-methylthioxanthon umgesetzt wird (bräunliches Oel), Ausbeute 22 %.

Analyse für $C_{17}H_{17}NOS$ (Molgewicht 283,39) :
berechnet : C 72,05 H 5,34 N 4,94 %
gefunden : C 72,41 H 5,50 N 4,82 %

## Beispiel 2

2-Brommethyl-thioxanthon wird nach der Vorschrift von G. Valiliu et al., Rev. Chim. (Bukarest), *19*, 561 (1968) hergestellt ; Smp. 193-196 °C.

Analyse :
berechnet : C 55,10 H 2,97 Br 26,18 %
gefunden : C 54,12 H 3,06 Br 26,09 %.

10 g (0,032 8 Mol) 2-Brommethylthioxanthon und 2,13 g (0,032 8 Mol) $NaN_3$ werden in 50 ml N,N-Dimethylformamid gelöst und 12 Stunden bei 50 °C gerührt. Der Verlauf der Reaktion wird mit IR-Spektroskopie verfolgt. Sie ist beendet, wenn keine weitere Zunahme der Bande bei 2 100 $cm^{-1}$ erfolgt. Man lässt abkühlen, versetzt mit Eiswasser, trennt das 2-Azidomethyl-thioxanthon durch Filtration ab und wäscht es mehrmals mit Wasser. Ausbeute : 6,80 g (77,5 d. Th.).

Smp : 121-123 °C Analyse :
berechnet : C 69,89 H 4,60 N 5,18 %
gefunden : C 68,91 H 4,40 N 5,35 %.

5 g 2-Azidomethylthioxanthon werden in 50 ml N,N-Dimethylformamid gelöst und mit 0,5 g Pd-Kohle in einem Autoklaven versetzt. Dann wird Wasserstoff eingeleitet. Die Reaktion wird mittels IR-Spektroskopie verfolgt. Sie ist beendet, wenn die Bande bei 2 100 $cm^{-1}$ verschwunden ist. Man verdünnt das Reaktionsgemisch mit N,N-Dimethylformamid, erhitzt auf 60 °C, filtriert, kühlt das Filtrat ab und versetzt mit Wasser. Die ausgefallenen Kristalle werden durch Filtration abgetrennt und getrocknet. Man erhält 4,3 g (95,3 % d. Th. 2-Aminomethylthioxanthon. Smp. 134-136 °C.

Analyse :
berechnet : C 69,69 H 5,81 %
gefunden : C 69,52 H 5,73 %

## Beispiel 3

2 g (0,016 6 Mol) 2-Aminomethylthioxanthon werden in 50 ml Aethylenglykolmonomethyläther mit 1,78 g eines Polyäthylenglykoldiglycidyläthers mit einem Epoxidäquivalent von 2,64 mAequv/g, 11,4 g Bisphenol A, 14,89 g Bisphenyl-A-diglycidyläther und 0,12 g N,N-Dimethylbenzylamin versetzt und zum

Rückfluss erhitzt. Nach 5 Stunden lässt man auf Raumtemperatur abkühlen und fällt das Polymere in Wasser.

Ausbeute : 25,4 g (93 % d. Th.). [η] = 0,14 dl/g (0,5 Gew.% in Tetrahydrofuran bei 25 °C). Tg = 56 °C. Schwefelgehalt : 1,65 % (berechnet 1,7 %).

## Beispiel 4

5 g (0,020 7 Mol) 2-Aminomethylthioxanthon und 17,55 g eines Polyaddukts aus Bisphenol A und Bisphenol-A-diglycidyläther mit Glycidylendgruppen und einem Epoxidäquivalentgewicht von 842,2 werden in 76 ml Aethylenglykolmonoäthyläther gelöst und 6 Stunden am Rückfluss gerührt. Durch Fällen in Wasser werden 16,8 g (74,5 % d. Th.) Polymer isoliert.

Gehalt an N : berechnet 1,29 % gefunden 1,26 %.

Glasumwandlungstemperatur = 77,24 °C.

## Beispiel 5

3 g (0,008 79 mMol) 3-(2-Amino-2-propyl)-thioxanthon-1-carbonsäureäthyester und 7,44 g eines Polyaddukts aus Bisphenol A und Bisphenol-A-diglycidyläther mit Glycidylendgruppen und einem Epoxidäquivalentgewicht von 842,2 werden in 50 ml Diäthylenglykoldimethyläther unter Stickstoff bei 160 °C 4 Std lang gerührt. Das Polymer wird anschliessend durch Fällen in Wasser isoliert. Ausbeute : 9,38 g (89,8 % d. Th.). Glasumwandlungstemperatur = 83,9 °C ; durchschnittliches Molekulargewicht = 4 600 Dalton (bestimmt durch Osmometrie).

## Beispiel 6

Zur Prüfung der Lichtempfindlichkeit werden jeweils 6 g der in der folgenden Tabelle angegebenen Polymeren in 20 ml N,N-Dimethylformamid gelöst und mit 110 mg Kupfer(II)acetat und 10 mg $CuBr_2$ versetzt. Diese Lösung wird mit einem Rakelstab auf eine Polyesterfolie aufgetragen (Nassfilmdicke 50 µm) und nach 60 Minuten Trocknen bei 50 °C in einem Umluftofen durch eine Maske (21-Step Sensitivity Guide der Fa. Stouffer) mit einer 5 kW Quecksilberhochdrucklampe belichtet. Das nach dem Belichten sichtbare Bild wird in einem Kupferbad der Zusammensetzung $CuSO_4 \cdot 5H_2O$ 12 g/l, Formaldehyd 8 g/l, NaOH 15 g/l, Natriumkaliumtartrat 14 g/l, Aethylendiamintetraessigsäure 20 g/l, Octylphenolpolyäthylenglykoläther 1 g/l (n ∞ l, Triton X 100® der Fa. Röhm + Haas) bei 49 °C zu einem metallischen, elektrisch leitenden Bild verstärkt. Die Resultate sind in der folgenden Tabelle angegeben.

Tabelle

| Polymer nach Bsp. Nr. | Belichtungs-zeit /Min. | Belichtungs-temperatur °C | letzte abgebildete Stufe |
|---|---|---|---|
| 3 | 3 | 90 | 5 |
| 4 | 3 | 80 | 6 |
| 5 | 3 | 90 | 4 |

**Patentansprüche**

1. Verbindungen der Formel I

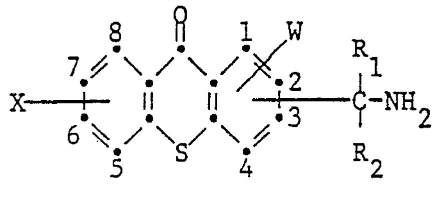

(I)

worin

X Wasserstoff, Chlor, Brom, $C_{1-4}$-Alkyl oder $C_{1-6}$-Alkoxy,

$R_1$ Wasserstoff, $C_{1-6}$-Alkyl, Phenyl, $-COOCH_3$ oder $-COOC_2H_5$ und

$R_2$ Wasserstoff, $C_{1-6}$-Alkyl, $-COOCH_3$ oder $-COOC_2H_5$ bedeuten oder
$R_1$ und $R_2$ zusammen $-(CH_2)_e-$ mit e = 4 oder 5 darstellen und
W Wasserstoff oder $-COOC_{1-4}$-Alkyl bedeutet.

2. Verbindungen der Formel I nach Anspruch 1, worin X Wasserstoff oder in 7-Stellung gebundenes Chlor oder Methyl bedeutet, die Gruppe $-C(R_1)(R_2)-NH_2$ in 2-, 3- oder 4-Stellung gebunden ist, $R_1$ und $R_2$ unabhängig voneinander Wasserstoff, Methyl, Aethyl, $-COOCH_3$ oder $-COOC_2H_5$ und W Wasserstoff, $-COOCH_3$ oder $-COOC_2H_5$ bedeuten.

3. Verbindungen der Formel I nach Anspruch 1, worin X Wasserstoff oder in 7-Stellung gebundenes Methyl ist, die Gruppe $-C(R_1)(R_2)-NH_2$ in 2- oder 3-Stellung gebunden ist, $R_1$ und $R_2$ je Wasserstoff oder Methyl und W Wasserstoff, $-COOCH_3$ oder $-COOC_2H_5$ bedeuten.

4. 2-Aminomethylthioxanthon, 3-(2-Amino-2-propyl)-thioxanthon, 3-(2-Amino-2-propyl)-7-methylthioxanthon und 3-(2-Amino-2-propyl)-thioxanthon-1-carbonsäureäthylester nach Anspruch 1.

5. Verfahren zur Herstellung von Verbindungen der Formel I nach Anspruch 1, dadurch gekennzeichnet, dass man

a) wenn $R_1$ ungleich Wasserstoff ist, eine Verbindung der Formel II

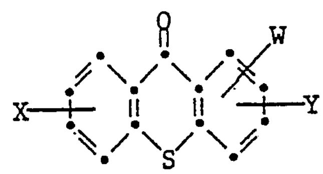

(II)

mit einer Verbindung der Formel III

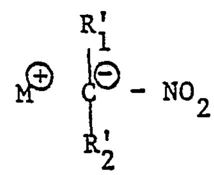

(III)

zu einer Verbindung der Formel IV

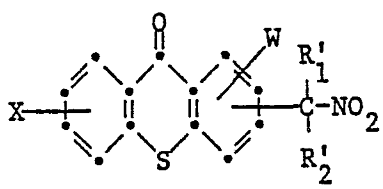

(IV)

umsetzt und die Verbindung der Formel IV zu einer Verbindung der Formel I reduziert, worin $R_1$ ungleich Wasserstoff ist

b) wenn $R_1$ und $R_2$ unabhängig voneinander $-COOCH_3$ oder $-COOC_2H_5$ bedeuten, eine Verbindung der Formel II, in Gegenwart einer Base mit einer Verbindung der Formel Va oder Vb

$$HC(R_1'')(R_2'')-NH_2 \qquad (Va)$$

oder

$$HC(R_1'')(R_2'')-NHCOCH_3 \qquad (Vb)$$

zu einer Verbindung der Formel Ia oder VI

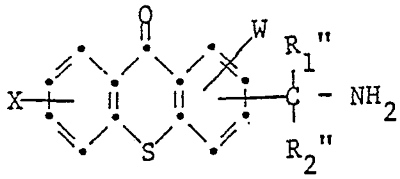

oder

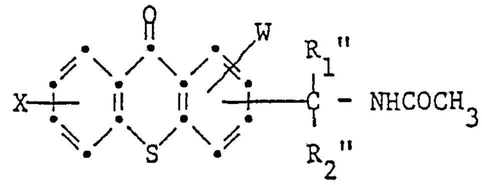

(Ia) (VI)

umsetzt, und die Verbindung der Formel VI zu einer Verbindung der Formel Ia hydrolysiert, worin $R_1''$ und $R_2''$ unabhängig voneinander —$COOCH_3$ oder —$COOC_2H_5$ darstellen, oder

c) wenn $R_1$ oder $R_1$ und $R_2$ Wasserstoff sind, eine Verbindung der Formel VII

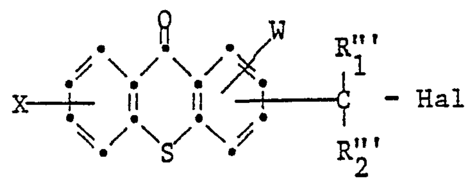

(VII)

mit einem Azid der Formel VIII

$$M_1^{q+}(N_3^-)_q \qquad (VIII)$$

zu einer Verbindung der Formel IX

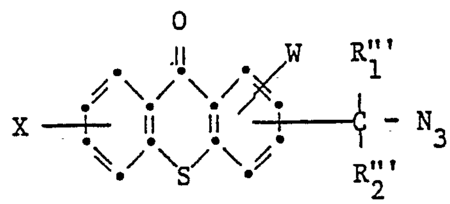

(IX)

umsetzt und die Verbindung der Formel IX zu einer Verbindung der Formel I reduziert, worin $R_1$ oder $R_2$ Wasserstoff sind, wobei

X und W die in Anspruch 1 angegebene Bedeutung haben,
Y Brom, Chlor, Fluor oder —$NO_2$ darstellt,
$R_1'$ und $R_2'$ dieselbe Bedeutung wie $R_1$ und $R_2$ haben, $R_1'$ aber ungleich Wasserstoff ist,
$R_1''$ und $R_2''$ unabhängig voneinander —$COOCH_3$ oder —$COOC_2H_5$ bedeuten,
$R_1'''$ Wasserstoff ist und $R_2'''$ dieselbe Bedeutung wie $R_2$ hat oder $R_1'''$ und $R_2'''$ beide Wasserstoff sind,
Hal ein Halogenatom,
$M^+$ das Kation einer organischen oder anorganischen Base,
q 1 oder 2 und
$M_1^+$ ein Alkalimetall-, Erdalkalimetall- oder quaternäres Ammoniumkation bedeuten.

6. Zu Kondensations- oder Additionsreaktionen befähigte lichtempfindliche und gegebenenfalls vernetzbare Stoffgemische, enthaltend

1) ein Thioxanthon der Formel I gemäss Anspruch 1
2) eine oder mehrere Verbindungen ausgewählt aus Di- bis Polyglycidyläthern von Phenol- und Kresolnovolacken und Verbindungen der Formeln XI bis XIII

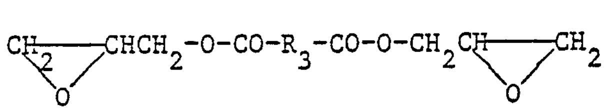

(XI)

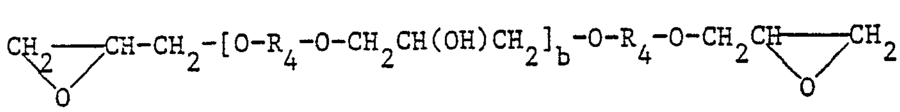

(XII)

und

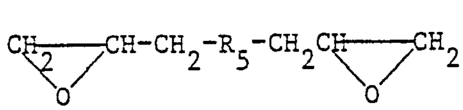

(XIII)

und gegebenenfalls Verbindungen der Formeln XIV, XV und/oder XVI

$$HO—R_6—O—[CO—R_3—CO—O—R_6—O]_a—H \quad (XIV)$$

$$HO—[Y_3—O]_o—Y_3—OH \quad (XV)$$

und/oder

$$HOOC—R_3—CO—[O—R_6—O—CO—R_3—CO]_a—OH \quad (XVI),$$

wobei der Anteil an Verbindungen der Formeln XIV, XV und/oder XVI höchstens 80 Mol.%, bezogen auf alle unter 2) genannten Reaktionskomponenten beträgt.

3) gegebenenfalls ein Vernetzungsmittel und

4) gegebenenfalls ein Salz eines Metalls der Gruppe Ib oder VIII des Periodischen Systems, worin

a eine Zahl von 1-100,

b eine Zahl von 0-150,

$R_3$ die direkte Bindung $—C_mH_{2m}—$ mit m = 2-12 oder Cyclohexylen, Cyclohexenylen, Phenylen oder Endomethylencyclohexenylen, die durch eine Methylgruppe substituiert sein können,

$R_4$ $—C_mH_{2m}—$ mit m = 2-12, Phenylen,

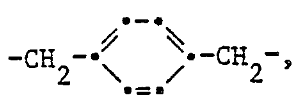, 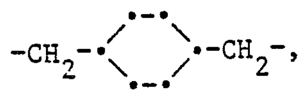,

eine Gruppe der Formeln

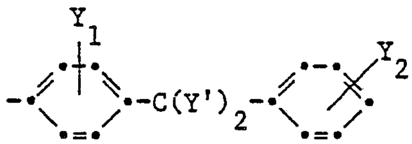

oder $—(Y_3O)_o—Y_3—$,

Y' Wasserstoff oder Methyl,

$Y_1$ und $Y_2$ unabhängig voneinander Wasserstoff, Chlor oder Brom,

$Y_3$ $—(CH_2)_2—$, $—CH_2CH(CH_3)—$ oder $—(CH_2)_4—$,

o eine Zahl von 1-50,

$R_5$

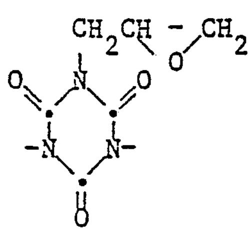, 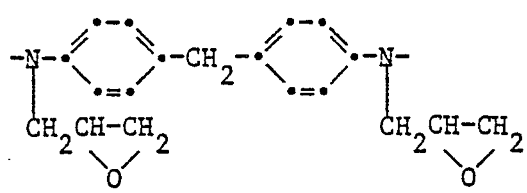

den Rest des Aethylenharnstoffs, 1,3-Propylenharnstoffs, 5,5-Dimethylhydantoins, 2-Hydroxyäthyl-5,5-dimethylhydantoins oder 2-Hydroxypropyl-5,5-dimethylhydantoins und

$R_6$ $—C_mH_{2m}—$ mit m = 2-12, $—(CH_2CH_2O)_r—CH_2CH_2—$ mit r = 1-40, $—CH(CH_3)CH_2OCH_2CH(CH_3)—$, $—CH_2—C(CH_3)_2—OCO—C(CH_3)_2CH_2—$, Cyclohexylen

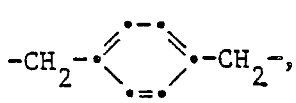, 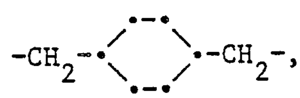,

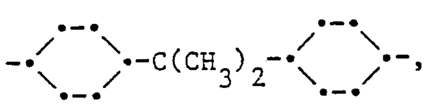, 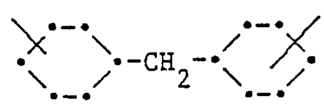

Naphthylen, Biphenylen oder gegebenenfalls durch eine Methyl-, Methoxy- oder Nitrogruppe substituiertes Phenylen bedeuten.

7. Stoffgemische nach Anspruch 6, enthaltend ein Thioxanthon der Formel I, eine oder mehrere definitionsgemässe Verbindungen mit Glycidylendgruppen und gegebenenfalls eine Verbindung der Formel XVI sowie gegebenenfalls ein Vernetzungsmittel und/oder ein definitionsgemässes Metallsalz, wobei X Wasserstoff oder in 7-Stellung gebundenes Chlor oder Methyl bedeutet, die Gruppe $—C(R_1)(R_2)—NH_2$ in 2-, 3- oder 4-Stellung gebunden ist, $R_1$ und $R_2$ unabhängig voneinander Wasserstoff, Methyl, Aethyl, $—COOCH_3$ oder $—COOC_2H_5$ und W Wasserstoff, $—COOCH_3$ oder $—COOC_2H_5$ bedeuten.

8. Stoffgemische nach Anspruch 6, enthaltend ein Thioxanthon der Formel I, worin X Wasserstoff oder in 7-Stellung gebundenes Methyl ist, die Gruppe $—C(R_1)(R_2)—NH_2$ in 2- oder 3-Stellung gebunden ist, $R_1$ und $R_2$ je Wasserstoff oder Methyl und W Wasserstoff, $—COOCH_3$ oder $—COOC_2H_5$ bedeuten, eine oder mehrere Verbindungen ausgewählt aus Di- und/oder Triglycidyläthern von Phenol- oder Kresolnovolacken, Triglycidylisocyanurat, Hexahydrophthalsäurediglycidylester, N,N'-Diglycidyl-5,5-dimethylhydantoin, N-Glycidyl-N'-2-oxyäthylglycidyl- und/oder N-Glycidyl-N'-oxypropylglycidyl-5,5-dimethylhydantoin und Verbindungen der Formeln A), B) und C)

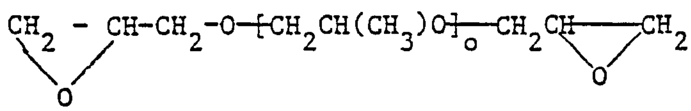
(A)

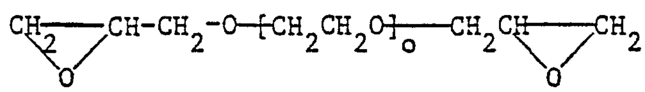
(B)

und

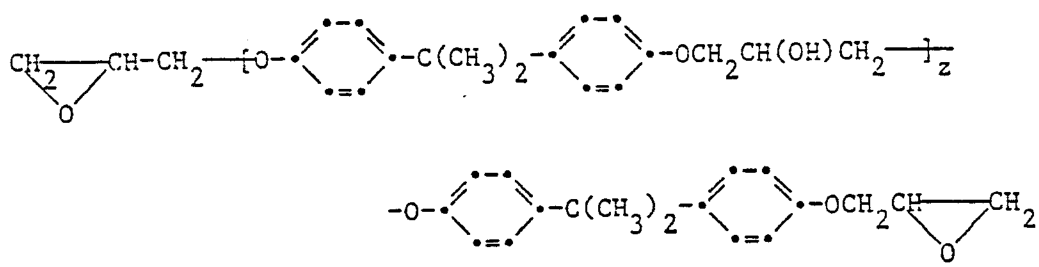
(C)

sowie gegebenenfalls ein Vernetzungsmittel und/oder ein definitionsgemässes Metallsalz, wobei o 2-40 und insbesondere 2-20 und z 0,1-13, vor allem 2-11, bedeuten.

9. Stoffgemische nach Anspruch 6, enthaltend als Verbindung der Formel I 2-Aminomethylthioxanthon, 3-(2-Amino-2-propyl)-thioxanthon, 3-(2-Amino-2-propyl)-7-methylthioxanthon oder 3-(2-Amino-2-propyl)-thioxanthon-1-carbonsäuremethylester, eine Verbindung der Formel A) oder B)

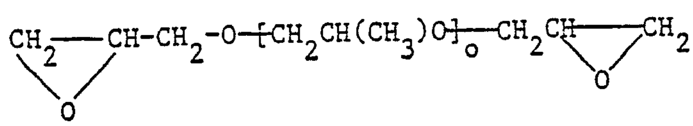
(A)

oder

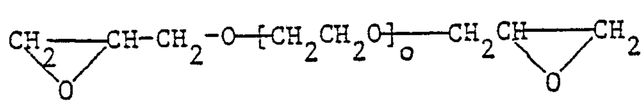
(B)

im Gemisch mit einer Verbindung der Formel C)

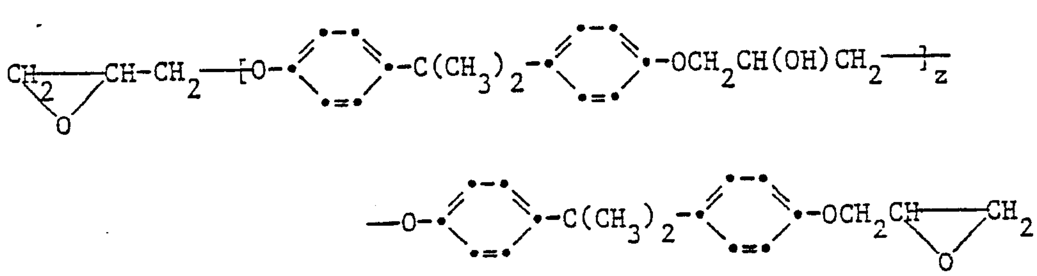
(C)

sowie gegebenenfalls ein Vernetzungsmittel und/oder ein definitionsgemässes Metallsalz, wobei o 2-20 und z 2-11 bedeuten.

10. Stoffgemische nach Anspruch 6, enthaltend als Verbindung der Formel I 2-Aminomethylthioxanthon, 3-(2-Amino-2-propyl)-thioxanthon, 3-(2-Amino-2-propyl)-7-methylthioxanthon oder 3-(2-Amino-2-propyl)-thioxanthon-1-carbonsäureäthylester, N,N'-Diglycidyl-5,5-dimethylhydantoin, N-Glycidyl-N'-2-oxyäthylglycidyl- und/oder N-Glycidyl-N'-2-oxypropylglycidyl-5,5-dimethylhydantoin im Gemisch mit einer Verbindung der Formel C)

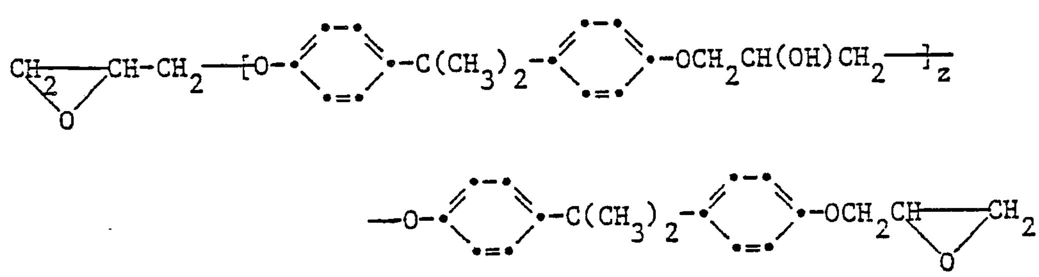

(C)

sowie gegebenenfalls ein Vernetzungsmittel und/oder ein definitionsgemässes Metallsalz, wobei o 2-20 und z 2-11 bedeuten.

11. Stoffgemische nach Anspruch 6, die ein Eisen-, Kobalt-, Nickel- oder Kupfersalz enthalten.

12. Stoffgemische nach Anspruch 6, die Kupfer(II)acetat oder ein Gemisch aus Kupfer(II)acetat und Kupfer(II)bromid enthalten.

13. Stoffgemische nach Anspruch 6, die als Vernetzungsmittel Hexahydrophthalsäureanhydrid oder Bisphenol A enthalten.

14. Lichtempfindliche, gegebenenfalls vernetzte Reaktionsprodukte, die dadurch erhältlich sind, dass man gegebenenfalls in Gegenwart eines Vernetzungsmittels eine Verbindung ausgewählt aus Di- bis Polyglycidyläthern von Phenol- und Kresolnovolacken und Verbindungen der Formeln XI bis XIII und gegebenenfalls Verbindungen der Formeln XIV, XV und/oder XVI nach Anspruch 1 zur Reaktion bringt, wobei für den Anteil an Verbindungen der Formeln XIV, XV und/oder XVI das im Anspruch 1 Angegebene gilt, und die erhaltenen Reaktionsprodukte gegebenenfalls anschliessend mindestens teilweise mit einem Salz eines Metalls der Gruppe Ib oder VIII des Periodischen Systems komplexiert.

15. Lichtempfindliche Reaktionsprodukte nach Anspruch 14, dadurch gekennzeichnet, dass man die erhaltenen Reaktionsprodukte mindestens teilweise mit einem Eisen-, Kobalt-, Nickel- oder Kupfersalz komplexiert.

16. Lichtempfindliche Reaktionsprodukte nach Anspruch 14, dadurch gekennzeichnet, dass man die erhaltenen Reaktionsprodukte mit Kupfer(II)acetat oder einem Gemisch aus Kupfer(II)acetat und Kupfer(II)bromid komplexiert.

17. Verwendung von Stoffgemischen nach Anspruch 6 zur Herstellung von elektrisch leitenden Ueberzügen oder Mustern durch Belichtung und anschliessende stromlose Metallabscheidung.

**Claims**

1. A compound of the formula I

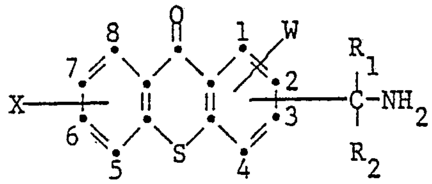

(I)

in which

X is hydrogen, chlorine, bromine, $C_{1-4}$-alkyl or $C_{1-6}$-alkoxy,

$R_1$ is hydrogen, $C_{1-6}$-alkyl, phenyl, $-COOCH_3$ or $-COOC_2H_5$ and

$R_2$ is hydrogen, $C_{1-6}$-alkyl, $-COOCH_3$ or $-COOC_2H_5$, or

$R_1$ and $R_2$ together are $-(CH_2)_e-$, where e is 4 or 5, and

W is hydrogen or $-COOC_{1-4}$-alkyl.

2. A compound of the formula I according to claim 1, in which X is hydrogen or chlorine or methyl bonded in the 7-position, the group $-C(R_1)(R_2)-NH_2$ is bonded in the 2-, 3- or 4-position, $R_1$ and $R_2$, each independently of the other, are hydrogen, methyl, ethyl, $-COOCH_3$ or $-COOC_2H_5$ and W is hydrogen, $-COOCH_3$ or $-COOC_2H_5$.

3. A compound of the formula I according to claim 1, in which X is hydrogen or methyl bonded in the 7-position, the group $—C(R_1)(R_2)—NH_2$ is bonded in the 2-, 3-position, $R_1$ and $R_2$ are each hydrogen or methyl and W is hydrogen, $—COOCH_3$ or $—COOC_2H_5$.

4. 2-Aminomethylthioxanthone, 3-(2-amino-2-propyl) thioxanthone, 3-(2-amino-2-propyl)-7-methylthioxanthone or ethyl 3-(2-amino-2-propyl) thioxanthone-1-carboxylate according to claim 1.

5. A process for the preparation of a compound of the formula I according to claim 1, which comprises

a) if $R_1$ is other than hydrogen, reacting a compound of the formula II

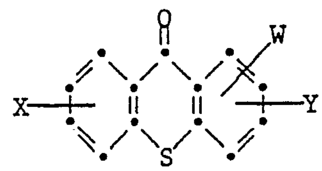

(II)

with a compound of the formula III

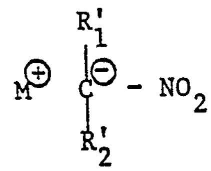

(III)

to give a compound of the formula IV

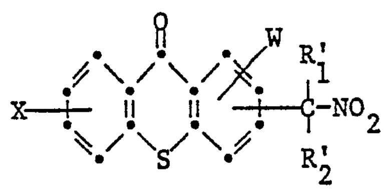

(IV)

and reducing the compound of the formula IV to a compound of the formula I in which $R_1$ is other than hydrogen,

b) if $R_1$ and $R_2$, each independently of the other, are $—COOH_3$ or $—COOC_2H_5$, reacting a compound of the formula II with a compound of the formula Va or Vb

$$HC(R_1'')(R_2'')—NH_2 \qquad (Va)$$

or

$$HC(R_1'')(R_2'')—NHCOCH_3 \qquad (Vb)$$

in the presence of a base, to give a compound of the formula Ia or VI

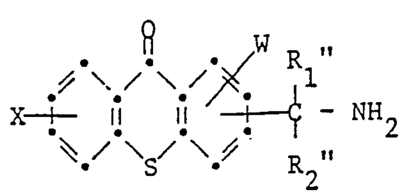

or

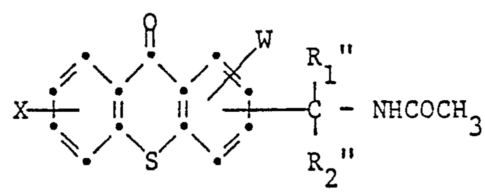

(Ia) (VI)

and hydrolysing the compound of the formula VI to give a compound of the formula Ia, in which $R_1''$ and $R_2''$, each independently of the other, are $—COOH_3$ or $—COOC_2H_5$, or

c) if $R_1$ or $R_1$ and $R_2$ are hydrogen, reacting a compound of the formula VII

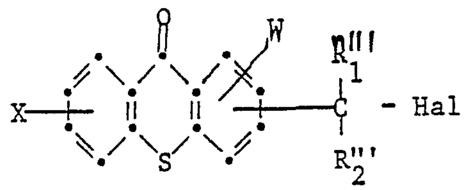

(VII)

with an azide of the formula VIII

$$M_1^{q+}(N_3^-)_q \qquad \text{(VIII)}$$

to give a compound of the formula IX

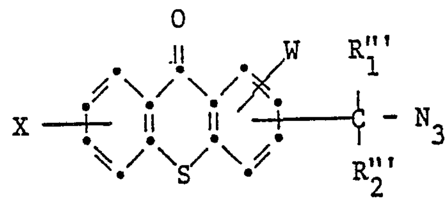

(IX)

and reducing the compound of the formula IX to a compound of the formula I, in which $R_1$ or $R_1$ and $R_2$ are hydrogen, in which formulae

X and W are as defined in claims 1,

Y is bromine, chlorine, fluorine or $—NO_2$,

$R_1'$ and $R_2'$ have the same meaning as $R_1$ and $R_2$, but $R_1'$ is other than hydrogen,

$R_1''$ and $R_2''$, each independently of the other, are $—COOCH_3$ or $—COOC_2H_5$,

$R_1'''$ is hydrogen and $R_2'''$ has the same meaning as $R_2$, or $R_1'''$ and $R_2'''$ are both hydrogen,

Hal is a halogen atom,

$M^+$ is the cation of an organic or inorganic base,

q is 1 or 2 and

$M_1^+$ is an alkali metal, alkaline earth metal or quaternary ammonium cation.

6. A photosensitive composition which is capable of undergoing condensation or addition reactions and may or may not be crosslinkable, containing

1) a thioxanthone of the formula I according to claim 1

2) one of more compounds selected from di- to polyglycidyl ethers of phenol novolaks and cresol novolaks and compounds of the formulae XI to XIII

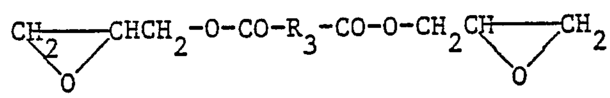

(XI)

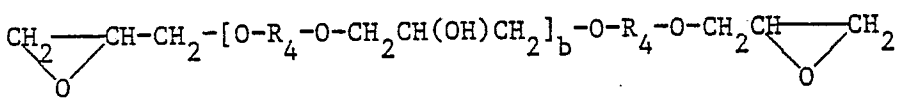

(XII)

and

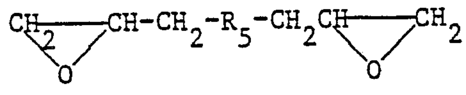

(XIII)

and, if appropriate, compounds of the formulae XIV, XV and/or XVI

$$HO—R_6—O—[CO—R_3—CO—O—R_6—O]_a—H \qquad \text{(XIV)}$$

$$HO—[Y_3—O]_o—Y_3—OH \qquad \text{(XV)}$$

and/or

$$HOOC—R_3—CO—[O—R_6—O—CO—R_3—CO]_a—OH \qquad \text{(XVI)}$$

the proportion of compounds of the formulae XIV, XV and/or XVI being at most 80 mole %, based on all the reactants mentioned unter 2),

3) if appropriate, a crosslinking agent and

4) if appropriate, a salt of a metal of group Ib or VIII of the Periodic Table, in which formulae

a is a number from 1 to 100,

b is a number from 0 to 150,

$R_3$ is a direct bond, $—C_mH_{2m}—$, where m = 2-12, or cyclohexylene, cyclohexenylene, phenylene or endomethylenecyclohexenylene, each of which can be substitured by a methyl group,

$R_4$ is $—C_mH_{2m}—$, where m = 2-12, phenylene,

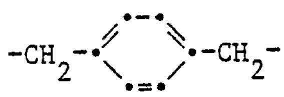, 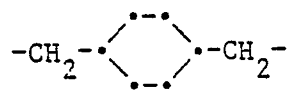,

or a group of the formula

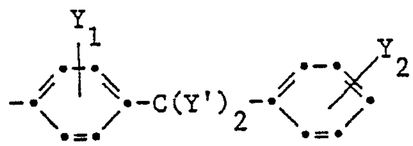

or $—(Y_3O)_o—Y_3—$

Y' is hydrogen or methyl,

$Y_1$ and $Y_2$, each independently of the other, are hydrogen, chlorine or bromine,

$Y_3$ is $—(CH_2)_2—$, $—CH_2CH(CH_3)—$ or $—(CH_2)_4—$,

o is a number from 1 to 50, $R_5$ is

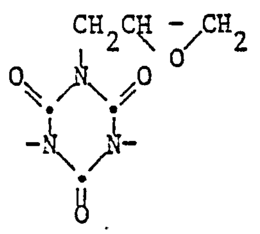, 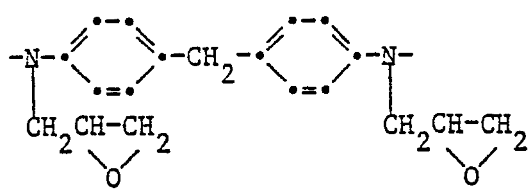

or the radical of ethylene-urea, 1,3-propylene-urea, 5,5-dimethylhydantoin, 2-hydroxyethyl-5,5-dimethylhydantoin or 2-hydroxypropyl-5,5-dimethylhydantoin and

$R_6$ is $—C_mH_{2m}—$, where m = 2-12, $—(CH_2CH_2O)_r—CH_2CH_2$, where r = 1-40, $—CH(CH_3)CH_2OCH_2CH(CH_3)—$, $—CH_2—C(CH_3)_2—OCO—C(CH_3)_2CH_2—$, cyclohexylene,

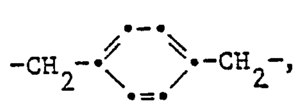, 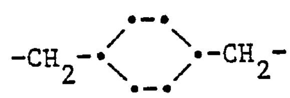,

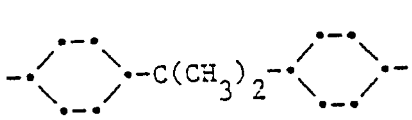, 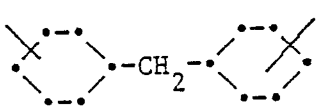

naphthylene or biphenylene, or phenylene which is unsubstituted or substituted by a methyl, methoxy or nitro group.

7. A composition according to claim 6, containing a thioxanthone of the formula I, one or more compounds of the type defined with glycidyl end groups and, if appropriate, a compound of the formula XVI, and, if appropriate, a crosslinking agent and/or a metal salt of the type defined, in which X is hydrogen or chlorine or methyl bonded in the 7-position, the group $—C(R_1)(R_2)—NH_2$ is bonded in the 2-, 3- or 4-position, $R_1$ and $R_2$, each independently of the other, are hydrogen, methyl, ethyl, $—COOCH_3$ or $—COOC_2H_5$ and W is hydrogen, $—COOCH_3$ or $—COOC_2H_5$.

8. A composition according to claim 6, containing a thioxanthone of the formula I in which X is hydrogen or methyl bonded in the 7-position, the group $—C(R_1)(R_2)—NH_2$ is bonded in the 2- or 3-position, $R_1$ and $R_2$ are each hydrogen or methyl and W is hydrogen, $—COOCH_3$ or $—COOC_2H_5$, one or more compounds selected from di- and/or triglycidyl ethers of phenol novolaks or cresol novolaks, triglycidylisocyanurate, diglycidyl hexahydrophthalate, N,N'-diglycidyl-5,5-dimethylhydantoin, N-glycidyl-N'-2-hydroxyethylglycidyl-5,5-dimethylhydantoin and/or N-glycidyl-N'-2-hydroxy-propylglycidyl-5,5-dimethylhydantoin and compounds of the formulae A), B) and C)

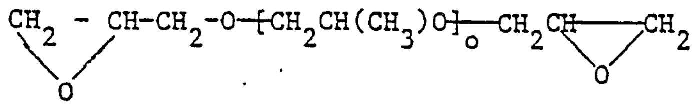 (A)

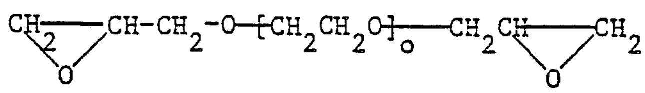 (B)

and

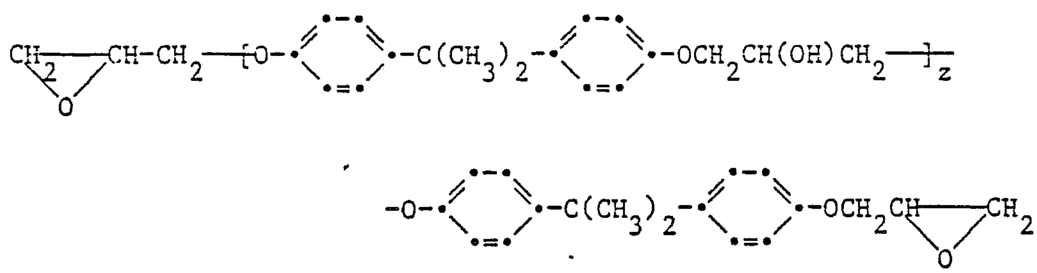 (C)

and, if appropriate, a crosslinking agent and/or a metal salt of the type defined, in which o is 2-40, in particular 2-20, and z is 0.1-13, in particular 2-11.

9. A composition according to claim 6, containing, as the compound of the formula 1, 2-aminomethylthioxanthone, 3-(2-amino-2-propyl)thioxanthone, 3-(2-amino-2-propyl)-7-methylthioxanthone or methyl 3-(2-amino-2-propyl)-thioxanthone-1-carboxylate, a compound of the formula A) or B)

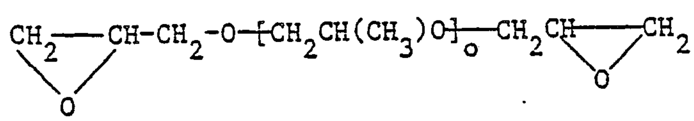 (A)

or

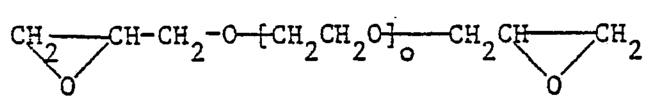 (B)

in admixture with a compound of the formula C

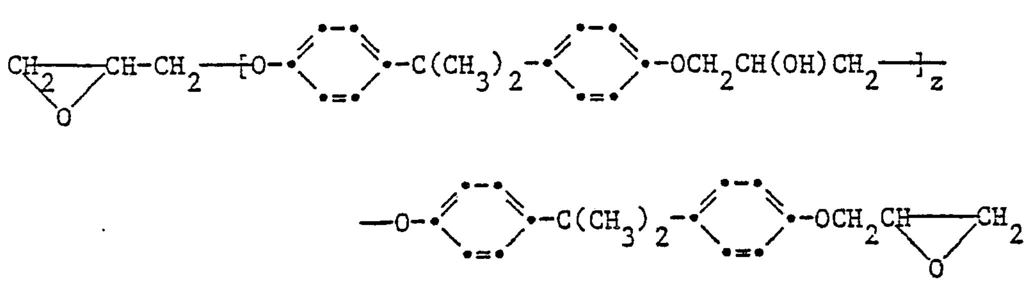 (C)

and, if appropriate, a crosslinking agent and/or a metal salt of the type defined, in which o is 2-20 and z is 2-11.

10. A composition according to claim 6, containing, as the compound of the formula I, 2-aminomethylthioxanthone, 3-(2-amino-2-propyl)thioxanthone, 3-(2-amino-2-propyl)-7-methylthioxanthone or ethyl 3-(2-amino-2-propyl)-thioxanthone-1-carboxylate, N,N'-diglycidyl-5,5-dimethylhydantoin, N-glycidyl-N'-2-hydroxyethylglycidyl-5,5-dimethylhydantoin and/or N-glycidyl-N'-2-hydroxypropylglycidyl-5,5-dimethylhydantoin, in admixture with a compound of the formula C)

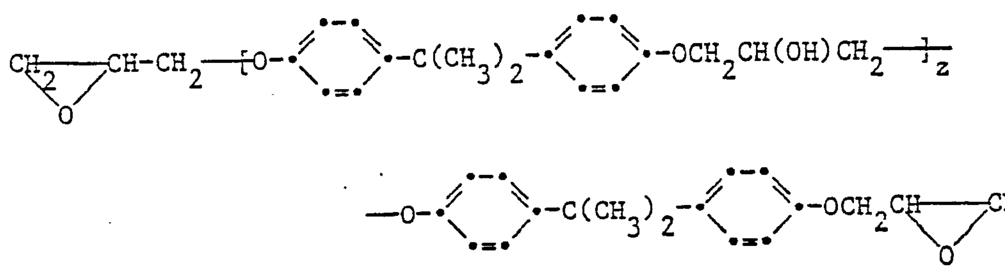 (C)

and, if appropriate, a crosslinking agent and/or a metal salt of the type defined, in which o is 2-20 and z is 2-11.

11. A composition according to claim 6, which contains an iron, cobalt, nickel or copper salt.

12. A composition according to claim 6, which contains copper(II) acetate or a mixture of copper(II) acetate and copper(II) bromide.

13. A composition according to claim 6, which contains hexahydrophthalic anhydride or bisphenol A as the crosslinking agent.

14. A photosensitive reaction product, which may or may not be crosslinked and can be obtained by reacting a compound of the formula I according to claim 1 with one or more compounds selected from di- to polyglycidyl ethers of phenol novolaks and cresol novolaks and compounds of the formulae XI et XIII and, if appropriate, compounds of the formulae XIV, XV and/or XVI according to claim 1, the proportion of compounds of the formulae XIV, XV and/or XVI being as defined in claim 1, in the presence or absence of a crosslinking agent, and, if appropriate, then at least partly complexing the resultant reaction product with a salt of a metal of group Ib or VIII of the Periodic Table.

15. A photosensitive reaction product according to claim 14, which is at least partly complexed with an iron, cobalt, nickel or copper salt.

16. A photosensitive reaction product according to claim 14, which is complexed with copper(II) acetate or a mixture of copper(II) acetate and copper(II) bromide.

17. Use of a composition according to claim 6 for the preparation of an electrically conductive coating or pattern by exposure to light and subsequent metal deposition without current.

**Revendications**

1. Composés répondant à la formule I

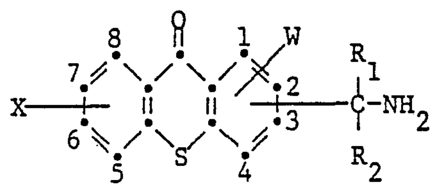 (I)

dans laquelle

X représente l'hydrogène, le chlore, le brome, un alkyle en $C_1$-$C_4$ ou un alcoxy en $C_1$-$C_6$,

$R_1$ représente l'hydrogène, un alkyle en $C_1$-$C_6$, un phényle, $-COOCH_3$ ou $-COOC_2H_5$,

$R_2$ représente l'hydrogène, un alkyle en $C_1$-$C_6$, $-COOCH_3$ ou $-COOC_2H_5$, ou encore

$R_1$ et $R_2$ forment ensemble un radical $-(CH_2)_e-$ dans lequel e désigne le nombre 4 ou le nombre 5, et

W représente l'hydrogène ou un radical alcoxycarbonyle dont la partie alcoxy contient de 1 à 4 atomes de carbone.

2. Composés de formule I selon la revendication 1 dans lesquels X représente l'hydrogène ou, en position 7, le chlore ou un méthyle, le radical $-C(R_1)(R_2)-NH_2$ occupe la position 2, 3 ou 4, $R_1$ et $R_2$ représentent chacun, indépendamment l'un de l'autre, l'hydrogène, un méthyle, un éthyle, $-COOCH_3$ ou $-COOC_2H_5$, et W représente l'hydrogène, $-COOCH_3$ ou $-COOC_2H_5$.

3. Composés de formule I selon la revendication 1 dans lesquels X représente l'hydrogène ou, en position 7, un méthyle, le radical $-C(R_1)(R_2)-NH_2$ se trouve en position 2 ou en position 3, $R_1$ et $R_2$ représentent chacun l'hydrogène ou un méthyle, et W représente l'hydrogène, $-COOCH_3$ ou $-COOC_2H_5$.

4. Composés selon la revendication 1, en l'espèce l'aminométhyl-2 thioxanthone, l'(amino-1 méthyl-1 éthyl)-3 thioxanthone, l'(amino-1 méthyl-1 éthyl)-3 méthyl-7 thioxanthone et l'(amino-1 méthyl-1 éthyl)-3 thioxanthonecarboxylate-1 d'éthyle.

5. Procédé de préparation de composés de formule I selon la revendication 1, procédé caractérisé en ce que :

a) lorsque $R_1$ ne représente pas l'hydrogène on fait réagir un composé de formule II

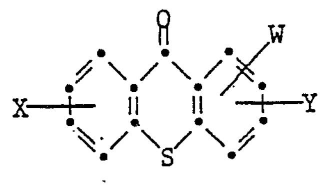

(II)

avec un composé de formule III

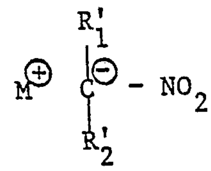

(III)

réaction qui conduit à un composé de formule IV

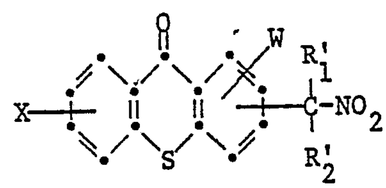

(IV)

et on réduit le composé de formule IV en un composé de formule I dans lequel $R_1$ ne représente pas l'hydrogène,

b) lorsque $R_1$ et $R_2$ représentent chacun, indépendamment l'un de l'autre, un radical $-COOCH_3$ ou $-COOC_2H_5$ on fait réagir un composé de formule II, en présence d'une base, avec un composé répondant à l'une des formules Va et Vb :

$$HC(R_1'')(R_2'')-NH_2 \quad (Va)$$

$$HC(R_1'')(R_2'')-NHCOCH_3 \quad (Vb)$$

de manière à obtenir un composé répondant à l'une des formules Ia et VI :

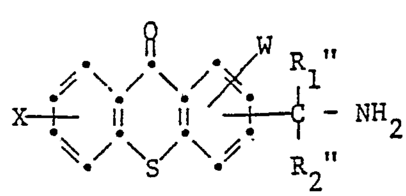

(Ia)

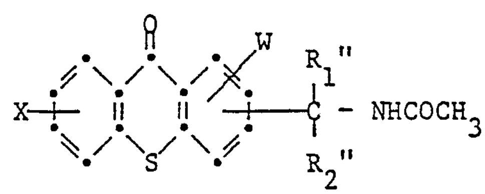

(VI)

et on hydrolyse le composé de formule VI pour le convertir en un composé de formule Ia, formules dans lesquelles $R_1''$ et $R_2''$ représentent chacun, indépendamment l'un de l'autre, un radical $-COOCH_3$ ou $-COOC_2H_5$, ou

c) lorsque $R_1$ ou $R_1$ et $R_2$ représentent l'hydrogène on fait réagir un composé de formule VII :

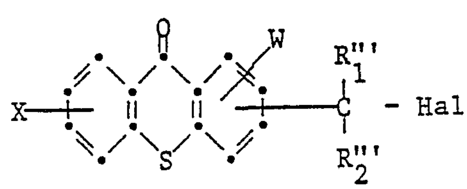

(VII)

avec un azoture de formule VIII :

$$M_1^{q+}(N_3^-)_q \qquad (VIII)$$

réaction qui conduit à un composé de formule IX :

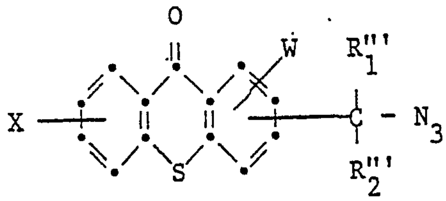

(IX)

et on réduit le composé de formule IX en un composé de formule I dans lequel $R_1$ ou $R_1$ et $R_2$ représentent l'hydrogène, les différents symboles présents dans les formules représentées ci-dessus ayant les significations suivantes :

X et W ont les significations données à la revendication 1,

Y représente le brome, le chlore, le fluor ou $—NO_2$,

$R_1'$ et $R_2'$ ont respectivement les mêmes significations que $R_1$ et $R_2$ mais $R_1'$ ne peut pas représenter l'hydrogène,

$R_1''$ et $R_2''$ représentent chacun, indépendamment l'un de l'autre, un radical $—COOCH_3$ ou $—COOC_2H_5$,

$R_1'''$ représente l'hydrogène,

$R_2'''$ a la même signification que $R_2$, ou

$R_1'''$ et $R_2'''$ représentent chacun l'hydrogène,

Hal représente un atome d'halogène,

$M^+$ représente le cation d'une base minérale ou organique,

q est égal à 1 ou à 2 et

$M_1^+$ représente un cation de métal alcalin ou de métal alcalino-terreux ou un cation d'ammonium quaternaire.

6. Mélanges photosensibles capables de donner des réactions de condensation ou d'addition et éventuellement réticulables qui contiennent :

1) une thioxanthone de formule I selon la revendication 1,

2) un ou plusieurs composés pris dans l'ensemble constitué par les éthers diglycidyliques et polyglycidyliques de novolaques du phénol et des crésols, les composés de formules XI à XIII :

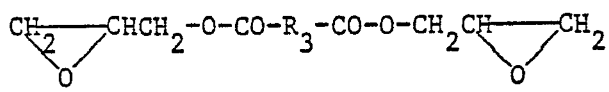

(XI)

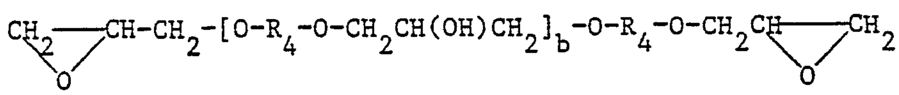

(XII)

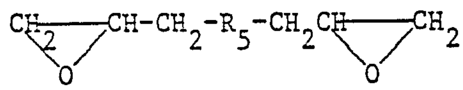

(XIII)

et, éventuellement, les composés de formules XIV, XV et XVI

$$HO—R_6—O—[CO—R_3—CO—O—R_6—O]_a—H \qquad (XIV)$$

$$HO—[Y_3—O]_c—Y_3—OH \qquad (XV)$$

$$HOOC—R_3—CO—[O—R_6—O—CO—R_3—CO]_a—OH \qquad (XVI)$$

la proportion des composés de formules XIV, XV et XVI étant d'au plus 80 % en moles par rapport à toutes les composantes réactionnelles mentionnés sous 2),

3) éventuellement un réticulant et

4) éventuellement un sel d'un métal du groupe Ib ou du groupe VIII de la classification périodique, les symboles présents dans les formules précédentes ayant les significations suivantes :

a représente un nombre de 1 à 100,

b représente un nombre de 0 à 150,

$R_3$ représente la liaison directe, un radical $—C_mH_{2m}—$ dans lequel m désigne un nombre de 2 à 12, ou un radical cyclohexylène, cyclohexénylène, phénylène ou endométhylène-cyclohexénylène, ce radical pouvant porter un radical méthyle,

$R_4$ représente un radical $—C_mH_{2m}—$ dans lequel m désigne un nombre de 2 à 12, un radical phénylène,

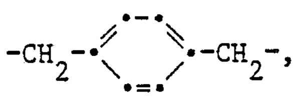

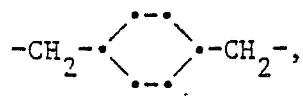

ou un radical répondant à l'une des formules :

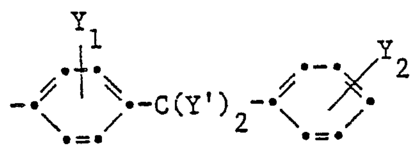

et $—(Y_3O)_o—Y_3—$

dans lesquelles :

Y' représente l'hydrogène ou un méthyle,

$Y_1$ et $Y_2$ représentent chacun, indépendamment l'un de l'autre, l'hydrogène, le chlore ou le brome,

$Y_3$ représente $—(CH_2)_2—$, $—CH_2CH(CH_3)—$ ou $—(CH_2)_4—$,

o représente un nombre de 1 à 50,

$R^5$ représente :

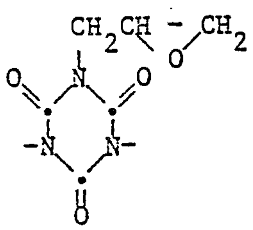

,

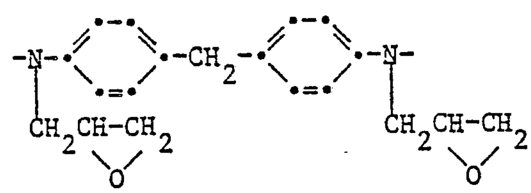

ou le radical de l'éthylène-urée, de la propylène-1,3 urée, de la diméthyl-5,5 hydantoïne, de l'(hydroxy-2 éthyl) diméthyl-5,5 hydantoïne ou de l'(hydroxy-2 propyl) diméthyl-5,5 hydantoïne, et

$R_6$ représente un radical $—C_mH_{2m}—$ dans lequel m désigne un nombre entier de 2 à 12, un radical $—(CH_2CH_2O)_r—CH_2CH_2—$ dans lequel r représente un nombre de 1 à 40, un radical $—CH(CH_3)CH_2OCH_2CH(CH_3)—$, $—CH_2—C(CH_3)_2—OCO—C(CH_3)_2CH_2—$, cyclohexylène,

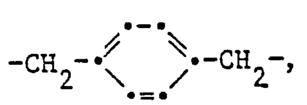

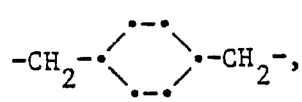

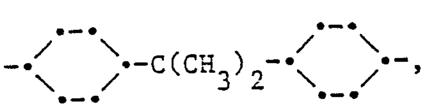

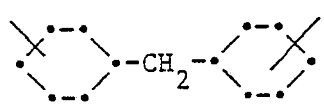

naphtylène ou biphénylylène ou un radical phénylène éventuellement porteur d'un radical méthyle, méthoxy ou nitro.

7. Mélanges selon la revendication 6 qui contiennent une thioxanthone de formule I dans laquelle X représente l'hydrogène ou, en position 7, le chlore ou un méthyle, le radical $—C(R_1)(R_2)—NH_2$ se trouve en position 2, 3 ou 4, $R_1$ et $R_2$ représentent chacun, indépendamment l'un de l'autre, l'hydrogène ou un radical méthyle, éthyle, $—COOCH_3$ ou $—COOC_2H_5$, et W représentent l'hydrogène ou un radical $—COOCH_3$ ou $—COOC_2H_5$, un ou plusieurs composés à radicaux glycidyles terminaux conformes à la définition, éventuellement un composé de formule XVI et, éventuellement, un réticulant et/ou un sel métallique conforme à la définition.

8. Mélanges selon la revendication 6 qui contiennent une thioxanthone de formule I dans laquelle X représente l'hydrogène ou, en position 7, un radical méthyle, le radical $—C(R_1)(R_2)—NH_2$ occupe la position 2 ou la position 3, $R_1$ et $R_2$ représentent chacun l'hydrogène ou un méthyle, et W représente

l'hydrogène ou un radical —$COOCH_3$ ou —$COOC_2H_5$, un ou plusieurs composés pris dans l'ensemble constitué par les éthers diglycidyliques et triglycidyliques de novolaques phénoliques et crésoliques, l'isocyanurate de triglycidyle, l'ester diglycidylique de l'acide hexahydrophtalique, la N,N'-diglycidyl diméthyl-5,5 hydantoïne, la N-glycidyl-N'-(glycidyloxy-2 éthyl) diméthyl-5,5 hydantoïne, la N-glycidyl-N'-(glycidyloxy-2 propyl) diméthyl-5,5 hydantoïne et les composés répondant aux formules A), B) et C)

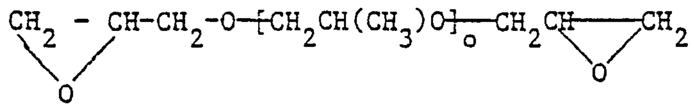
(A)

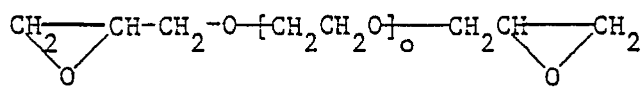
(B)

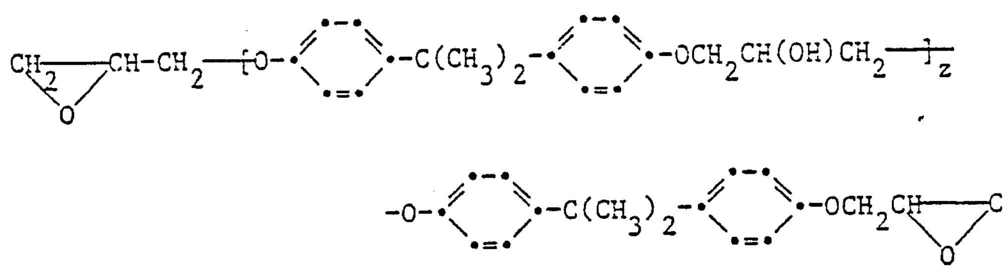
(C)

(où o désigne un nombre de 2 à 40, plus spécialement de 2 à 20, et z un nombre de 0,1 à 13, plus spécialement de 2 à 11), et, le cas échéant, un réticulant et/ou un sel métallique conforme à la définition.

9. Mélanges selon la revendication 6 qui contiennent comme composé de formule I, l'aminométhyl-2 thioxanthone, l'(amino-1 méthyl-1 éthyl)-3 thioxanthone, l'(amino-1 méthyl-1 éthyl)-3 méthyl-7 thioxanthone ou l'(amino-1 méthyl-1 éthyl)-3 thioxanthone-carboxylate-1 de méthyle, un composé répondant à la formule A) ou la formule B)

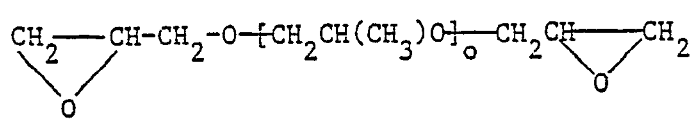
(A)

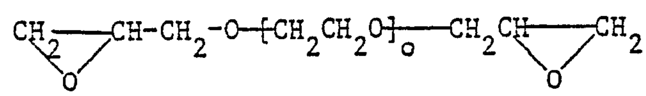
(B)

en mélange avec un composé de formule C)

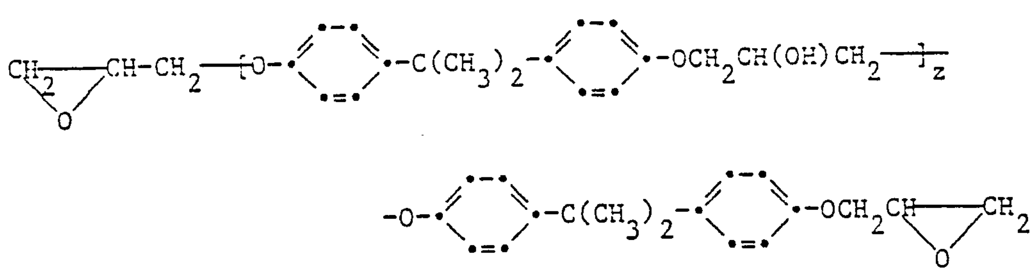
(C)

où o désigne un nombre de 2 à 20 et z un nombre de 2 à 11, et éventuellement un réticulant et/ou un sel métallique conforme à la définition.

10. Mélanges selon la revendication 6 qui contiennent comme composé de formule I, l'aminométhyl-2 thioxanthone, l'(amino-1 méthyl-1 éthyl)-3 thioxanthone, l'(amino-1 méthyl-1 éthyl)-3 méthyl-7 thioxanthone ou l'(amino-1 méthyl-1 éthyl)-3 thioxanthone-carboxylate-1 d'éthyle, la N,N'-diglycidyl diméthyl-5,5 hydantoïne, la N-glycidyl-N'-(glycidyloxy-2 éthyl) diméthyl-5,5 hydantoïne et/ou la N-glycidyl-N'-(glycidyloxy-2 propyl) diméthyl-5,5 hydantoïne en mélange avec un composé de formule C)

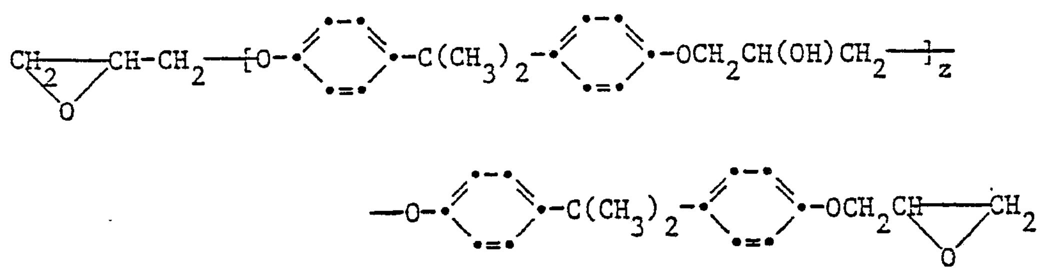

(C)

dans laquelle Z désigne un nombre de 2 à 11 et o un nombre de 2 à 20, et, éventuellement, un réticulant et/ou un sel métallique conforme à la définition.

11. Mélange selon la revendication 6 qui contiennent un sel de fer, de cobalt, de nickel ou de cuivre.

12. Mélanges selon la revendication 6 qui contiennent de l'acétate de cuivre(II) ou un mélange d'acétate de cuivre(II) et de bromure de cuivre(II).

13. Mélanges selon la revendication 6 qui contiennent, comme réticulant, de l'anhydride hexahydrophtalique ou du bisphénol A.

14. Produits réactionnels photosensibles, éventuellement réticulés, que l'on peut obtenir en faisant réagir, éventuellement en présence d'un réticulant, un composé de formule I selon la revendication 1 avec un ou plusieurs composés pris dans l'ensemble constitué par les éthers diglycidyliques et polyglycidyliques de novolaques phénoliques et crésoliques, les composés de formules XI à XIII et éventuellement les composés de formules XIV, XV et/ou XVI selon la revendication 1, la proportion des composés de formules XIV, XV et/ou XVI devant satisfaire à la condition énoncée à la revendication 1, et ensuite en complexant éventuellement les produits réactionnels obtenus, au moins partiellement, avec un sel d'un métal du groupe Ib ou VIII de la classification périodique.

15. Produits réactionnels photosensibles selon la revendication 14, caractérisée en ce qu'on complexe les produits réactionnels obtenus, au moins partiellement, avec un sel de fer, de cobalt, de nickel ou de cuivre.

16. Produits réactionnels photosensibles selon la revendication 14, caractérisés en ce qu'on complexe les produits réactionnels obtenus avec de l'acétate de cuivre(II) ou avec un mélange d'acétate de cuivre(II) et de bromure de cuivre(II).

17. Application de mélanges selon la revendication 6 à la fabrication de revêtements ou de dessins électroconducteurs, par exposition à un rayonnement, suivie d'un dépôt non électrolytique de métal.